# EUROPEAN PATENT SPECIFICATION

(11) **EP 2 773 765 B1**
(45) Date of publication and mention of the grant of the patent: **20.07.2016**
(21) Application number: 12846175.3
(22) Date of filing: 05.11.2012
(51) Int. Cl.: C12N 15/82, C12N 1/21, A01H 5/00

(54) **METHODS USING ACYL-COENZYME A-BINDING PROTEINS TO ENHANCE DROUGHT TOLERANCE IN GENETICALLY MODIFIED PLANTS**
VERFAHREN MIT ACYL-KOENZYM-A-BINDENDEN PROTEINEN ZUR VERBESSERUNG DER TOLERANZ GEGENÜBER TROCKENHEIT BEI GENMANIPULIERTEN PFLANZEN
PROCÉDÉS À L'AIDE DE PROTÉINES DE LIAISON À L'ACYL-COENZYME A POUR AMÉLIORER LA TOLÉRANCE À LA SÉCHERESSE DANS DES PLANTES GÉNÉTIQUEMENT MODIFIÉES

(30) Priority: 03.11.2011 US 201161555287 P
(43) Date of publication of application: 10.09.2014
(73) Proprietor: Versitech Limited, Hong Kong (CN)
(72) Inventor: CHYE, MeeLen, Hong Kong (CN); DU, ZhiYan, Hong Kong (CN); CHEN, MoXian, Hong Kong (CN)
(74) Representative: Isarpatent
(86) International application number: PCT/CN2012/084081
(87) International publication number: WO 2013/064119

(56) References cited:
- WO-A1-2008/113163
- CN-A- 1 768 575
- CN-A- 1 847 402
- US-A1- 2008 289 252
- GAO WEI ET AL: "Arabidopsis thaliana acyl-CoA-binding protein ACBP2 interacts with heavy-metal-binding farnesylated protein AtFP6", NEW PHYTOLOGIST, vol. 181, no. 1, 2009, pages 89-102, XP002737918, ISSN: 0028-646X
- HONG-YE LI ET AL: "Arabidopsis Acyl-CoA-Binding Protein ACBP2 Interacts With an Ethylene-Responsive Element-Binding Protein, AtEBP, via its Ankyrin Repeats", PLANT MOLECULAR BIOLOGY, KLUWER ACADEMIC PUBLISHERS, DORDRECHT, NL, vol. 54, no. 2, 1 January 2004 (2004-01-01), pages 233-243, XP019262456, ISSN: 1573-5028, DOI: 10.1023/B:PLAN.0000028790.75090.AB
- KOJIMA ET AL: "The effects of down-regulating expression of Arabidopsis thaliana membrane-associated acyl-CoA binding protein 2 on acyl-lipid composition", PLANT SCIENCE, ELSEVIER IRELAND LTD, IE, vol. 172, no. 1, 11 November 2006 (2006-11-11), pages 36-44, XP005760180, ISSN: 0168-9452, DOI: 10.1016/J.PLANTSCI.2006.07.009
- DU ZHI-YAN ET AL: "Arabidopsis acyl-CoA-binding proteins ACBP1 and ACBP2 show different roles in freezing stress.", PLANT SIGNALING & BEHAVIOR MAY 2010, vol. 5, no. 5, May 2010 (2010-05), pages 607-609, XP002737919, ISSN: 1559-2324
- CHEN QIN-FANG ET AL: "The Arabidopsis acbp1acbp2 double mutant lacking acyl-CoA-binding proteins ACBP1 and ACBP2 is embryo lethal", NEW PHYTOLOGIST, vol. 186, no. 4, 2010, pages 843-855, XP002737920, ISSN: 0028-646X
- DU ZHI-YAN ET AL: "Overexpression of Arabidopsis acyl-CoA-binding protein ACBP2 enhances drought tolerance", PLANT CELL AND ENVIRONMENT, vol. 36, no. 2, February 2013 (2013-02), pages 300-314, XP002737921,
- XIAO, SHI ET AL.: 'New roles for acyl-CoA-binding proteins (ACBPs) in plant development stress responses and lipid metabolism.' PROGRESS IN LIPID RESEARCH. vol. 50, 07 December 2010, pages 141 - 151, XP028166398
- GAO, WEI ET AL.: 'Acyl-CoA-binding protein 2 binds lysophospholipase 2 and lysoPC to promote tolerance to cadmium-inducted oxidative stress in transgenic Arabidopsis.' THE PLANT XP055149058

## Description

### CROSS-REFERENCE TO RELATED APPLICATIONS

This application claims benefit of and priority to U.S.S.N. 61/555,287, filed on November 3, 2011,.

### REFERENCE TO SEQUENCE LISTING

The Sequence Listing was submitted November 2, 2012as a text file named "UHK_00404_ST25.txt," was created on November 2, 2012, and has a size of 17,888 bytes.

### FIELD OF THE INVENTION

This disclosure generally relates to genetically modified plants and vectors for conferring drought tolerance to plants.

### BACKGROUND OF THE INVENTION

Drought stress is one of the biggest environmental threats to agriculture and human beings. Drought is a major stress to plants and it occurs when the total transpiration rate exceeds water uptake in plant cells (Ingram and Bartels, Annu Rev Plant Physiol Plant Mol Biol., 47:377-403, (1996)). Crops are especially susceptible to drought during flowering, and without flowers there would be no fruit and seeds (grain) which form the harvest in a majority of crops. Global warming further aggravates the problems related to drought. Thus, there is a need to identify genes that confer drought tolerance to create transgenic plants (especially crops) with improved ability to survive water deficit stress.
WO2008/113163, US 2008/289252 and Gao Wei et al., New Phytologist, vol. 181, no.1,, pages 89 - 102 describe the transgenic over-expression of acyl-Coenzyme A-binding proteins in plants and seeds. The enhancement of draught tolerance is not described.

It is an object of the present invention to provide vectors that confer drought tolerance to transgenic plants and plant material.

It is also an object of the present invention to provide transgenic plant and plant material with enhanced drought tolerance, and method of making them.

It is still another object of the present invention to provide methods for screening for genes that confer drought tolerance to plants.

### SUMMARY OF THE INVENTION

Provided are transgenic plants and plant material to be used for the improvement of drought tolerance and vectors for producing them. It has been discovered that expressing Arabidopsis thaliana acyl-Coenzyme A-binding protein 2 (ACBP2) in plants improves drought tolerance relative to unmodified plants. Plant parts, include for example, fruits, leaves, tubers, seeds, flowers, stems, roots, and all other anatomical parts of the modified plant wherein the ACBP2 polypeptide is expressed are also provided to be used for the improvement of drought tolerance. In specific embodiments, the transformed plants are transgenic or transplastomic Arabidopsis, tomato, tobacco, cotton, corn, and rice plants. In a specific non-limiting example, drought tolerance in a plant can be measured by the ability to survive without water for at least 15 days.

Plant transformation vectors (not claimed per se) for improving draught tolerance in plants include a nucleic acid sequence encoding an ACBP2 polypeptide or a functional fragment or variant of ACBP2 as defined herein and in the claims. In some embodiments, the vectors comprise a promoter, operably linked to a sequence encoding an ACBP2 polypeptide or a functional fragment or variant of ACBP2 as defined herein and in the claims, and a terminator, and/or other regulatory elements. The promoter can be constitutive, inducible or tissue specific. In other embodiments, the vector can be designed so that it will be expressed under the control of a plant's own endogenous promoter. The vectors may encode more than one ACBP2 polypeptide or a functional fragment or variant of ACBP2 as an operon. The vectors described herein include plant plastid transformation vectors or nuclear transformation vectors.

Also provided is a method of producing modified plants or plant cells with drought tolerance. The method includes transforming a plant or plant cell with the vectors described herein, which comprise an ACBP2-encoding polynucleotide. In some embodiments, a nuclear transformation vector is used to cause expression of one or more ACBP2s or variants thereof having at least 90% amino acid identity to SEQ ID NO: 6 and having Arabidopsis thaliana acyl-Coenzme A-binding protein 2 activity, conveying similar drought tolerance as the Arabidopsis ACBP2 polypeptides. In other embodiments, a plastid transformation vector is used to cause expression of one or more ACBP2s or variants thereof having at least 90% amino acid identity to SEQ ID NO: 6 and having Arabidopsis thaliana acyl-Coenzme A-binding protein 2 activity conveying similar drought tolerance as the Arabidopsis ACBP2 polypeptides. Such nuclear and plastid transformation vectors can be used alone or in conjunction with each other or with other recombinant vectors that can enhance the drought tolerance of plants transformed therewith.

Also provided is a method for screening for ACBP2-like sequences or variants having at least 90% amino acid identity to SEQ ID NO: 6 and having Arabidopsis thaliana acyl-Coenzme A-binding protein 2 activity, which confer drought tolerance to plants. The method includes introducing an exogenous nucleic acid into a plant host cell which exhibits growth inhibition in drought conditions, to form a plant test cell, where relief of growth inhibition indicates ACBP2-like ability to confer growth tolerance. In some embodiments, the exogenous nucleic acid is mutated prior to being introduced into the plant host cell. In other embodiments, the exogenous nucleic acid is a synthetic nucleic acid encoding a variant of ACBP2 as defined herein and in the claims.
Specifically, the following main aspects are covered by the invention.
Use of *Arabidopsis thaliana* acyl-Coenzme A-binding protein 2 (ACBP2) according to SEQ ID NO: 6 or peptide sequences having at least 90% amino acid identity to SEQ ID NO: 6 and having Arabidopsis thaliana acyl-Coenzme A-binding protein 2 activity to enhance drought tolerance relative to an unmodified plant in a transgenic plant or a transgenic plant cell genetically engineered to express ACBP2 according to SEQ ID NO: 6 or peptide sequences having at least 90% amino acid identity to SEQ ID NO: 6 and having Arabidopsis thaliana acyl-Coenzme A-binding protein 2 activity in an amount effective to enhance drought tolerance relative to an unmodified plant.
A method of obtaining enhanced drought tolerance in a plant or plant cell comprising: genetically engineering the plant or plant cell to express *Arabidopsis thaliana* acyl-Coenzme A-binding protein 2 (ACBP2) according to SEQ ID NO: 6 or peptide sequences having at least 90% amino acid identity to SEQ ID NO: 6 and having Arabidopsis thaliana acyl-Coenzme A-binding protein 2 activity in an amount effective to provide drought tolerance relative to a non-genetically engineered plant.
A method of obtaining a plant part having drought tolerance, comprising: obtaining a plant part genetically modified to express *Arabidopsis thaliana* acyl-Coenzme A-binding protein 2 (ACBP2) according to SEQ ID NO: 6 or peptide sequences having at least 90% amino acid identity to SEQ ID NO: 6 and having Arabidopsis thaliana acyl-Coenzme A-binding protein 2 activity; and growing the plant part under conditions where it is exposed to drought stress sufficient to be growth-inhibiting to a native plant of the same type.
A method of screening for functional variants of Arabidopsis thaliana acyl-Coenzme A-binding protein 2 (ACBP2) according to SEQ ID NO: 6 or peptide sequences having at least 90% amino acid identity to SEQ ID NO: 6 wherein the functional variants have at least 90% amino acid identity to SEQ ID NO: 6 and have Arabidopsis thaliana acyl-Coenzme A-binding protein 2 activity, comprising: obtaining a plant cell genetically modified to express a candidate ACBP2 variant; growing the plant cell under conditions of drought stress for a duration that is sufficient to be growth-inhibiting to a native plant cell of the same type; observing whether the plant cell exhibits a reduction in growth inhibition; and, if so, identifying the candidate ACBP2 variant as functional.

### BRIEF DESCRIPTION OF THE DRAWINGS

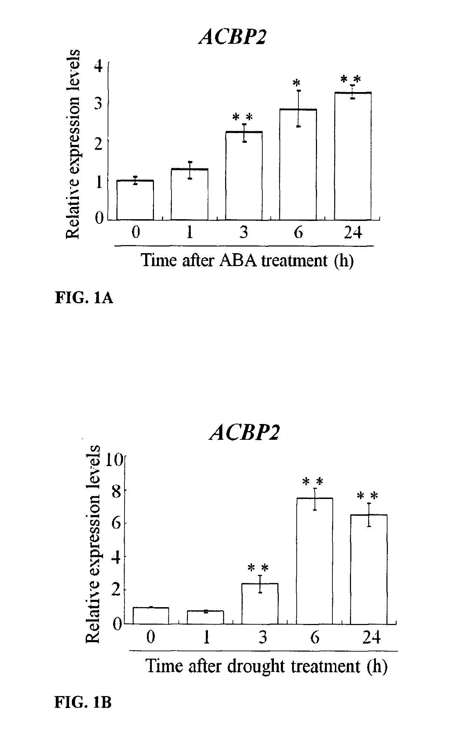

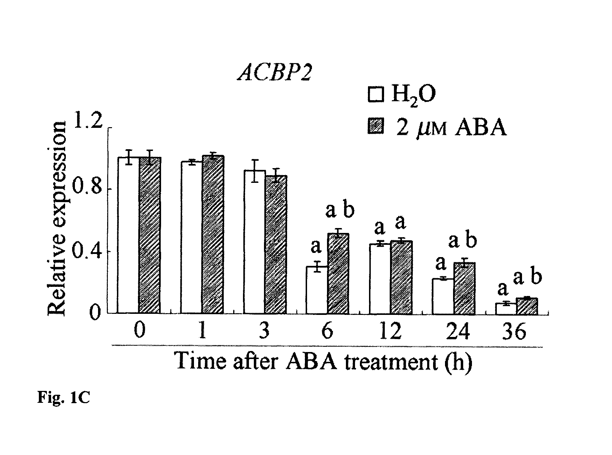

**Figs. 1A** and **1B** show the expression of ACBP2 in response to ABA **(****Fig. 1A****)** and drought treatment **(****Fig. 1B**) in 12-day-old Arabidopsis seedlings. Fig. 1A values are means ± SD. ** P < 0.01, * P < 0.05, n = 4. **Fig. 1B** values are means ± SD. ** P < 0.01, n = 4. **Fig. 1C** shows the expression of *ACBP2* during seed germination at the indicated times (hour) with or without ABA (2 µM) treatment. a, indicates significant differences in comparison to H2O control at 0 h; b, indicates significant differences at a similar time (*P* < 0.05, *n* = 5).

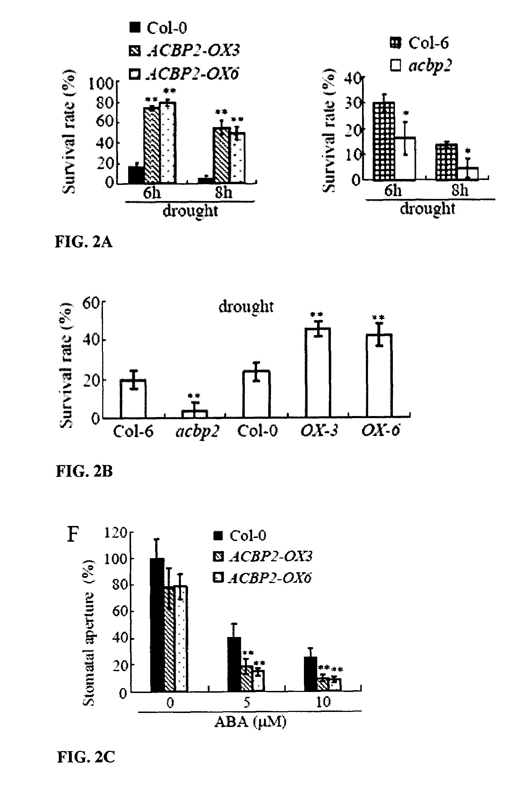

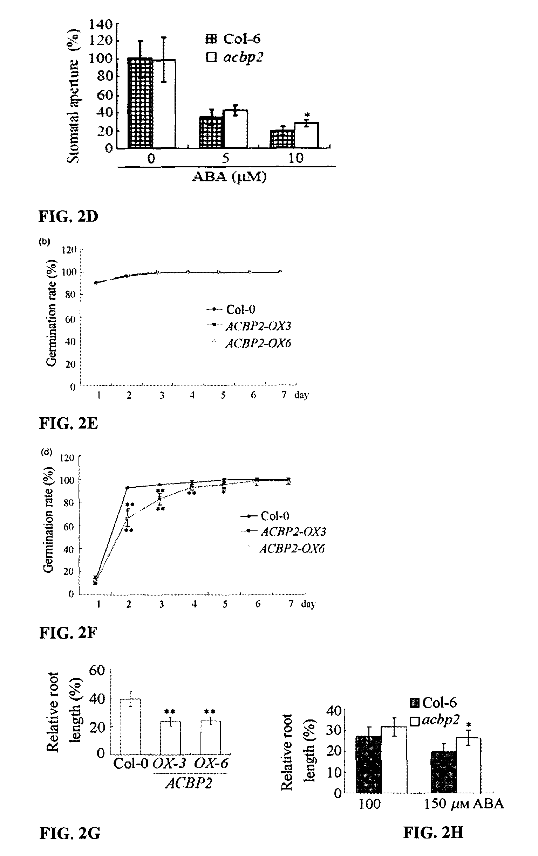

**Figs. 2A-2D** show tolerance of ACBP2-OXs to drought treatment compared to wild type plants. **Fig. 2A** is a bar graph showing the survival of Col-0 compared to ACBP2-OX3 and ACBP2-OX6, and acbp2 compared to the Col-0. ** P < 0.01, * P < 0.05. Values are means ± SD (n = 4). **Fig. 2B** is a bar graph showing the survival of Wild-type (Col-0 and Col-6), acbp2 mutant, ACBP2-OX3 and ACBP2-OX6 plants following a 15 d in drought treatment. ** P < 0.01. Values are means ± SD (n = 4, 30 plants from each genotype were tested in each of four experiments). **Fig. 2C** shows the effect of ABA on stomatal closure in the wild type (Col-0) and ACBP2-OXs leaves. **Fig. 2D** shows the effect of ABA on stomatal closure in the wild type (Col-6) and acbp2 mutant Leaves. Bar = 20 µm. Values are the means ± SD. The asterisks indicate statistically significant differences. ** P < 0.01, * P < 0.05, n = 3 (30 guard cells of each line were examined and repeated for three times). **Fig. 2E** **- 2F** show germination rates of ACBP2-OX3 and ACBP2-OX6 in the absence **(****Fig. 2E****)** and presence **(****Fig. 2F****)** of ABA compared to control, Col-0. Values are means ± SD, *n* = 3. ** *P* < 0.01, * *P* < 0.05. **Fig. 2G** shows relative root length in ACBP2-OX3 and ACBP2-OX6 compared to control, following ABA treatment. ** *P* < 0.01.Values are means ±SD, *n* = 3 (50 seedlings per genotype were measured and the experiment was repeated 3 times). **Fig. 2H** shows relative root length following 100 µm or 150 µm treatment in acbp2 mutants, compared to control. * *P* < 0.05.Values are means ±SD, *n* = 3 (50 seedlings per genotype were measured and the experiment was repeated three times).

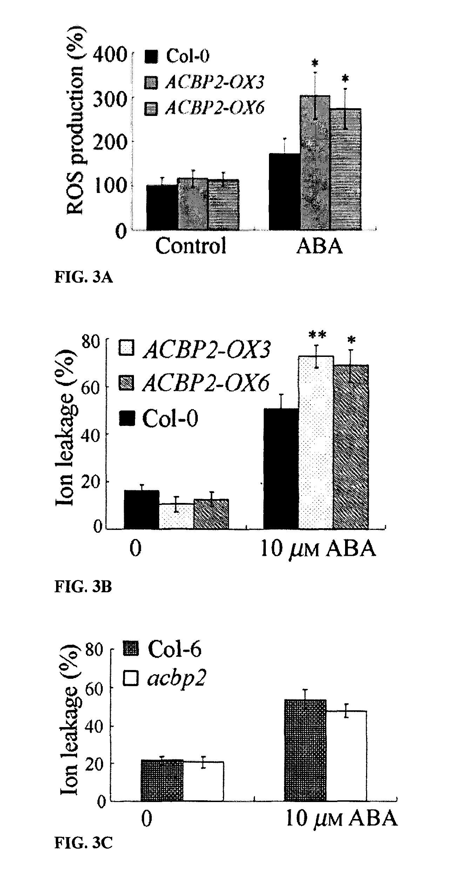

**Fig. 3A** is a bar graph showing relative fluorescence intensity indicating ABA-induced ROS production (%) in guard cells, in response to overexpression of ACBP2. ROS production in wild-type (Col-0, control group) equals 100%. *P<0.05. **Fig. 3B** shows ion leakage of detached leaves from 5-week-old wild-type (Col-0) and *ACBP2-OX* (*OX-3* and *OX-6*) plants incubated with or without 10 µm ABA for 3 d. **Fig. 3C** shows ion leakage of detached leaves from 5-week-old wild-type (Col-6) and *acbp2* mutant incubated with or without 10 µm ABA for 3 d.

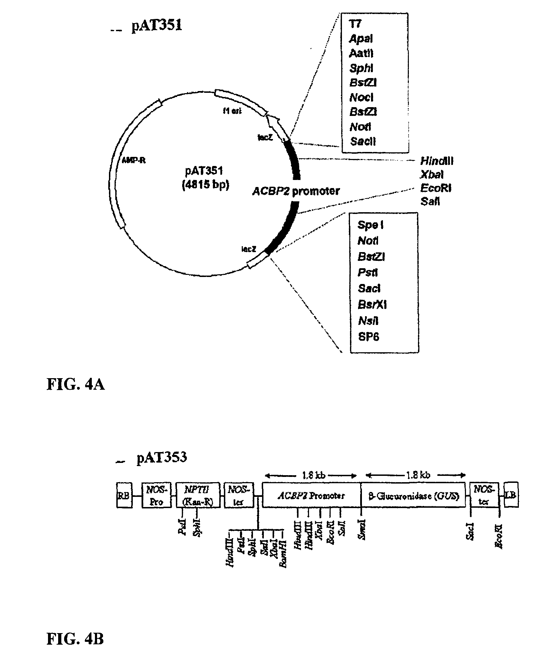

**Figs. 4A** and **4B** show the restriction map of transformation vector pAT351 and pAT353. **Fig. 4A** shows plasmid pAT351. Fig. 4B shows plasmid pAT353.

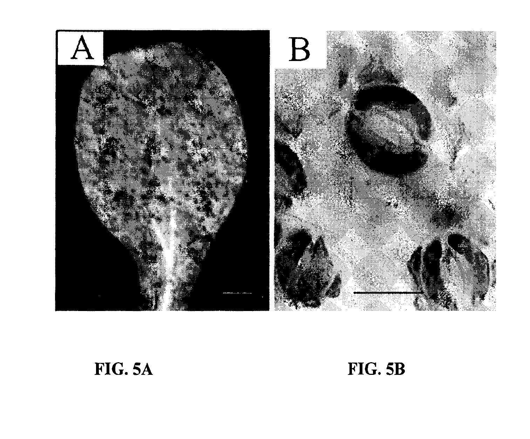

Figs. 5A and 5B show ACBP2pro:GUS expression in leaves **(****Fig. 5A**) and guard cells **(****Fig. 5B****)** of transgenic Arabidopsis as evidenced by Histochemical GUS assays were carried out using 5 bromo-4-chloro-3-indolyl-b-D-glucuronide (X-Gluc), Scale bars = 1 mm (FIG. 5A) and 20 µm (FIG. 5B).

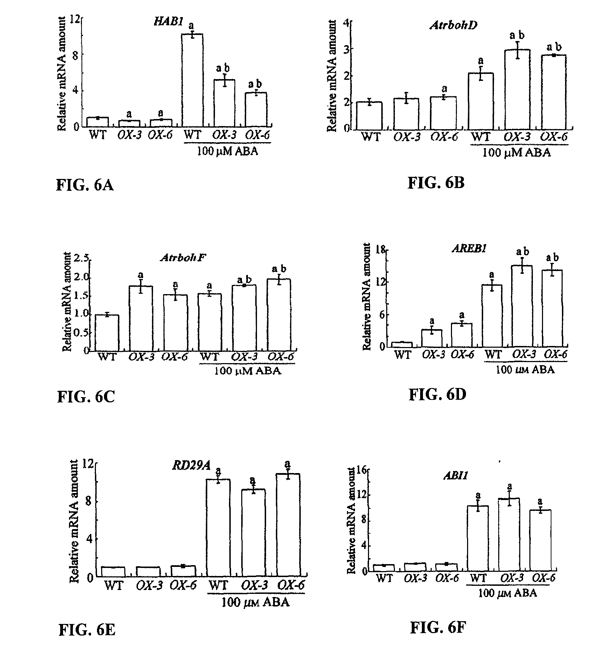

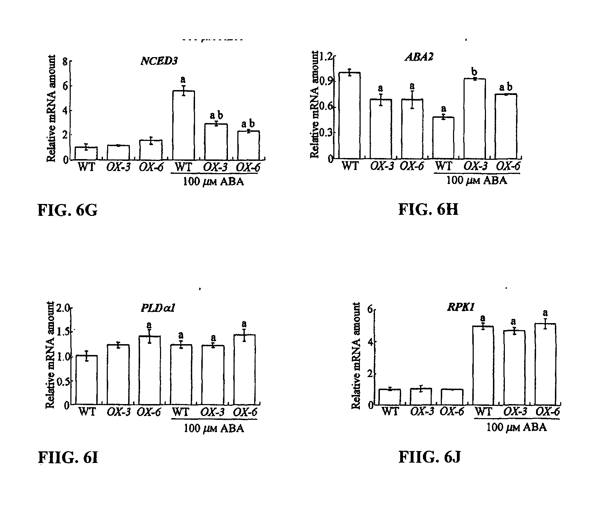

Figs. 6A-6C are bar graphs which show the effect of overexpression of ACBP2 on HAB1 **(****FIG. 6A****),** AtrbohD **(****FIG. 6B****),** AtrbohF. **(****FIG. 6C****),** *AREB1* **(****Fig. 6D****),** *RD29A* **(****Fig. 6E****),** *ABI1* **(****Fig. 6F****),** *NCED3* **(****Fig. 6G****),** *ABA2* **(****Fig. 6H****),** *PLDα1* **(****Fig. 6I****)** and *RPK1* **(****Fig. 6J****).** a indicates significant difference compared with the untreated wild type; b indicates significant difference compared with the ABA-treated wild type (P < 0.05, n = 5).

### DETAILED DESCRIPTION OF THE INVENTION

Genetically modified plants and progeny thereof expressing the acyl-CoA-binding protein, ACBP2, exemplified herein by the Arabidopsis ACBP2 protein, exhibit improved drought tolerance as compared to non-modified plants. Thus, the overexpression of ACBP2 polypeptide in crop plants can help them withstand drought stress and extend cultivation zones.

### I. DEFINITIONS

"ACBP2" is used herein to mean Arabidopsis thaliana acyl-Coenzyme A-binding protein 2 and functional variants thereof (polynucleotides or polypeptides, as indicated by the context) that can convey improved drought tolerance to the host in which they are expressed. In a specific non-limiting example, drought tolerance in a plant can be measured by the ability to survive without water for at least 15 days.

"ACBP2-OXs" is used herein to mean transgenic Arabidopsis thaliana overexpressing ACBP2 polypeptide.

"ACBP2-like polypeptide" as used herein includes polypeptides sharing at least 77% sequence identity to ACBP2 that convey improved drought tolerance to the host cell, including variants of ACBP2 described below.

ACBP2-like polypeptide, ACBP variants and ACBP2 homologs as used herein refer to polypeptides, which like ACBP2, can down regulate the negative regulator of ABA-mediated drought regulatory proteins (for example, HAB1) and upregulate the positive regulators like AtrbohD and AtrbohF.

"Chemically synthesized," as related to a sequence of DNA, means that the component nucleotides were assembled in vitro.

"Construct" as used herein refers to a recombinant nucleic acid, generally recombinant DNA, which has been generated for the purpose of the expression of a specific nucleotide sequence(s), or is to be used in the construction of other recombinant nucleotide sequences.

"Cotyledon" refers to the embryonic first leaves of a seedling.

"DNA regulatory sequences," "control elements," and "regulatory elements," are used interchangeably herein, refer to transcriptional and translational control sequences, such as promoters, enhancers, polyadenylation signals, terminators, protein degradation signals, and the like, that provide for and/or regulate expression of a coding sequence and/or production of an encoded polypeptide in a host cell.

"Endogenous nucleic acid" as used herein refers to a nucleic acid that is normally found in and/or produced by a given bacterium, organism, or cell in nature. An "endogenous nucleic acid" is also referred to as a "native nucleic acid" or a nucleic acid that is "native" to a given bacterium, organism, or cell.

"Exogenous nucleic acid" as used herein refers to a nucleic acid that is not normally or naturally found in and/or produced by a given bacterium, organism, or cell in nature.

"Heterologous nucleic acid," as used herein, refers to a nucleic acid wherein at least one of the following is true: (a) the nucleic acid is foreign ("exogenous" i.e., not naturally found in) a given host microorganism or host cell; (b) the nucleic acid comprises a nucleotide sequence that is naturally found in e.g., is "endogenous to" a given host microorganism or host cell (e.g., the nucleic acid comprises a nucleotide sequence endogenous to the host microorganism or host cell); however, in the context of a heterologous nucleic acid, the same nucleotide sequence as found endogenously is produced in an unnatural (e.g., greater than expected or greater than naturally found) amount in the cell, or a nucleic acid comprising a nucleotide sequence that differs in sequence from the endogenous nucleotide sequence but encodes the same protein (having the same or substantially the same amino acid sequence) as found endogenously is produced in an unnatural (e.g., greater than expected or greater than naturally found) amount in the cell; (c) the nucleic acid comprises two or more nucleotide sequences that are not found in the same relationship to each other in nature, e.g., the nucleic acid is recombinant. An example of a heterologous nucleic acid is a nucleotide sequence encoding an ACBP2 operably linked to a transcriptional control element (for example, a 5 promoter) to which an endogenous (naturally-occurring) ACBP2 coding sequence is not normally operably linked. Another example of a heterologous nucleic acid is a high copy number plasmid comprising a nucleotide sequence encoding an ACBP2. Another example of a heterologous nucleic acid is a nucleic acid encoding an ACBP2, where a host cell that does not normally produce ACBP2 is genetically modified with the nucleic acid encoding ACBP2; because ACBP2-encoding nucleic acids are not naturally found in the host cell, the nucleic acid is heterologous to the genetically modified host cell.

"Host cell," as used herein, denotes an in vivo or in vitro eukaryotic cell, a prokaryotic cell, or a cell from a multicellular organism (for example, a cell line) cultured as a unicellular entity, which eukaryotic or prokaryotic cells can be, or have been, used as recipients for a nucleic acid (for example, an expression vector that comprises a nucleotide sequence encoding one or more gene products such as ACBPs), and includes the progeny of the original cell which has been genetically modified by the nucleic acid. It is understood that the progeny of a single cell may not necessarily be completely identical in morphology or in genomic or total DNA complement as the original parent, due to natural, accidental, or deliberate mutation. A "recombinant host cell" (also referred to as a "genetically modified host cell") is a host cell into which has been introduced a heterologous nucleic acid, e.g., an expression vector.

"Isolated" is meant to describe a polynucleotide, a polypeptide, or a cell that is in an environment different from that in which the polynucleotide, the polypeptide, or the cell naturally occurs. An isolated genetically modified host cell may be present in a mixed population of genetically modified host cells.

"Naturally-occurring" or "native" as used herein as applied to a nucleic acid, a cell, or an organism, refers to a nucleic acid, cell, or organism that is found in nature. For example, a polypeptide or polynucleotide sequence that is present in an organism (including viruses) that can be isolated from a source in nature and which has not been intentionally modified by a human in the laboratory is naturally occurring, and "wild-type" plants are naturally occurring.

"Modified plant or plant parts" as used herein refers to a plant or plant part, whether it is attached or detached from the whole plant. It also includes progeny of the modified plant or plant parts that are produced through sexual or asexual reproduction.

"Operably linked" refers to a juxtaposition wherein the components so described are in a relationship permitting them to function in their intended manner. For instance, a promoter is operably linked to a coding sequence if the promoter affects its transcription or expression.

"Operon" and "single transcription unit" are used herein interchangeably to refer to two or more contiguous coding regions (nucleotide sequences that encode a gene product such as an RNA or a protein) that are coordinately regulated by one or more controlling elements (e.g., a promoter). As used herein, the term "gene product" refers to RNA encoded by DNA (or vice versa) or protein that is encoded by an RNA or DNA, where a gene will typically comprise one or more nucleotide sequences that encode a protein, and may also include introns and other non-coding nucleotide sequences.

"Peptide," "polypeptide," and "protein" are used interchangeably herein, and refer to a polymeric form of amino acids of any length, which can include coded and non-coded amino acids, chemically or biochemically modified or derivatized amino acids, and polypeptides having modified peptide backbones.

Percent "sequence identity" of a polypeptide or polynucleotide to another polynucleotide or polypeptide, meaning that, when aligned, that percentage of bases or amino acids are the same, and in the same relative position, when comparing the two sequences.

"Plant cell culture" refers to cultures of plant units such as, for example, protoplasts, cell culture cells, cells in plant tissues, pollen, pollen tubes, ovules, embryo sacs, zygotes and embryos at various stages of development.

"Plant material" refers to leaves, stems, roots, flowers or flower parts, fruits, pollen, egg cells, zygotes, seeds, cuttings, cell or tissue cultures, or any other part or product of a plant.

"Plant tissue" refers to a group of plant cells organized into a structural and functional unit. Any tissue of a plant, whether in a plant or in culture, is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

"Polynucleotide" and "nucleic acid," are used interchangeably herein, and refer to a polymeric form of nucleotides of any length, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases.

"Progeny" includes the immediate and all subsequent generations of offspring traceable to a parent.

"Recombinant," as used herein, means that a particular nucleic acid (DNA or RNA) is the product of various combinations of cloning, restriction, and/or ligation steps resulting in a construct having a structural coding or non-coding sequence distinguishable from endogenous nucleic acids found in natural systems. Generally, DNA sequences encoding the structural coding sequence can be assembled from cDNA fragments and short oligonucleotide linkers, or from a series of synthetic oligonucleotides, to provide a synthetic nucleic acid which is capable of being expressed from a recombinant transcriptional unit contained in a cell or in a cell-free transcription and translation system. Such sequences can be provided in the form of an open reading frame uninterrupted by internal non-translated sequences, or introns, which are typically present in eukaryotic genes. Genomic DNA comprising the relevant sequences can also be used in the formation of a recombinant gene or transcriptional unit. Sequences of non-translated DNA may be present 5' or 3' from the open reading frame, where such sequences do not interfere with manipulation or expression of the coding regions, and may indeed act to modulate production of a desired product by various mechanisms (see "DNA regulatory sequences", below). Thus, for example, the term "recombinant" polynucleotide or nucleic acid refers to one which is not naturally occurring, for example, is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques. Such is usually done to replace a codon with a redundant codon encoding the same or a conservative amino acid, while typically introducing or removing a sequence recognition site. Alternatively, it is performed to join together nucleic acid segments of desired functions to generate a desired combination of functions.

"Transformation" or "transformed" are used interchangeably herein with "genetic modification" or "genetically modified" and refer to a permanent or transient genetic change induced in a cell following introduction of new nucleic acid (i.e., DNA exogenous to the cell). Genetic change ("modification") can be accomplished either by incorporation of the new DNA into the genome of the host cell, or by transient or stable maintenance of the new DNA as an episomal element. Where the cell is a eukaryotic cell, a permanent genetic change is generally achieved by introduction of the DNA into the genome of the cell or into a plastome of the cell. In prokaryotic cells, permanent changes can be introduced into the chromosome or via extrachromosomal elements such as plasmids, plastids, and expression vectors, which may contain one or more selectable markers to aid in their maintenance in the recombinant host cell.

"Transformation vectors and "expression cassettes" are used herein interchangeably.

"Synthetic nucleic acids" can be assembled from oligonucleotide building blocks that are chemically synthesized using procedures known to those skilled in the art. These building blocks are ligated and annealed to form gene segments which are then enzymatically assembled to construct the entire gene.

"Variant" as used herein refers either to a naturally occurring genetic mutant of ACBP2 or a recombinantly prepared variation of ACBP2, each of which contain one or more mutations in its DNA. The term "variant" may also refer to either a naturally occurring variation of a given peptide or a recombinantly prepared variation of a given peptide or protein in which one or more amino acid residues have been modified by amino acid substitution, addition, or deletion.

### II. VECTORS FOR CONFERRING DROUGHT RESISTANCE

In Arabidopsis, a total of six forms of acyl-Coenzyme A-binding proteins (ACBPs) have been identified and designated as ACBP1 to ACBP6 (Xiao, et al., Plant J., 54:141-151 (2008)), ranging from 10 to 73.1 kD (Leung, et al., Plant Mol Biol., 55:297-309 (2004)). Membrane-associated ACBP1 and ACBP2 are subcellularly localized to the ER and plasma membrane (Chye, et al., Plant J., 18:205-214 (1999); Li and Chye, Plant Mol Biol., 51:483-492 (2003)), ACBP3 is extracellularly-targeted (Leung, et al., Planta, 223:871-881 (2006)) and kelch-motif-containing ACBP4 and ACBP5 (Leung, et al., Plant Mol Biol., 55:297-309 (2004)), as well as ACBP6 are localized in the cytosol (Chen et al., Plant Physiol., 148: 304-315, 2008). Domains that potentially mediate protein-protein interactions, ankyrin repeats (ACBP1 and ACBP2) and kelch motifs (ACBP4 and ACBP5) (Leung et al., Plant Mol Biol., 55: 297-309, 2004; Li and Chye, Plant Mol Biol., 54: 233-243, 2004), are evident in the larger ACBPs. Arabidopsis ACBPs have been implicated in various stress responses, such as freezing (Chen et al., Plant Physiol., 148:304-315 (2008); Du et al., Plant Physiol., 152:1585-1597 (2010)) and pathogen resistance (Xiao and Chye, Plant Physiol., 156:2069-2081 (2011)). The Examples described herein show that the overexpression of ACBP2 confers drought tolerance in plants.

The plant transformation vectors/expression cassettes provided herein (but not claimed per se) include a nucleic acid sequence encoding an ACBP2 polypeptide or a functional variant of ACBP2 thereof. The vector can optionally also include a promoter, operably linked to the coding sequence, and a terminator, and/or other regulatory elements. In other embodiments, the vector can be designed to introduce the heterologous polypeptide so that it will be expressed under the control of a plant's own endogenous promoter. The plant transformation vectors preferably include a transcription initiation or transcriptional control region(s) the coding region for the protein of interest, and a transcriptional termination region. Transcriptional control regions include those that provide for over-expression of the protein of interest in the genetically modified host cell; those that provide for inducible expression, such that when an inducing agent is added to the culture medium, transcription of the coding region of the protein of interest is induced or increased to a higher level than prior to induction.

In one embodiment, the construct contains operatively linked in the 5' to 3' direction, a promoter; one or more nucleic acid sequence encoding an ACBP2 or a functional variant or fragment of ACBP2; and a 3' polyadenylation signal. In embodiments where the construct contains more than one ACBP2 or a functional variant of ACBP2 thereof expressed as an operon, the nucleotide sequences can be operably linked to the same promoter. Alternatively, the nucleotide sequences may be under the control of different promoters.

Several plant transformation vector options are available, including those described in "Gene Transfer to Plants" (Potrykus, et al., eds.) Springer-Verlag Berlin Heidelberg New York (1995); "Transgenic Plants: A Production System for Industrial and Pharmaceutical Proteins" (Owen, et al., eds.) John Wiley & Sons Ltd. England (1996); and "Methods in Plant Molecular Biology: A Laboratory Course Manual" (Maliga, et al. eds.) Cold Spring Laboratory Press, New York (1995). Plant transformation vectors generally include one or more coding sequences of interest under the transcriptional control of 5' and 3' regulatory sequences, including a promoter, a transcription termination and/or polyadenylation signal, and a selectable or screenable marker gene. For the expression of two or more polypeptides from a single transcript, additional RNA processing signals and ribozyme sequences can be engineered into the construct (U.S. Pat. No.5,519,164). This approach has the advantage of locating multiple transgenes in a single locus, which is advantageous in subsequent plant breeding efforts.

For direct expression of transgenes from the plastid genome, a vector to transform the plant plastid chromosome by homologous recombination is used in which case it is possible to take advantage of the prokaryotic nature of the plastid genome and insert a number of transgenes as an operon. Examples are described in U.S. Pat. No. 5,545,818 to McBride et al. WO 2010/061186 describes an alternative method for introducing genes into the plastid chromosome using an adapted endogenous cellular process for the transfer of RNAs from the cytoplasm to the plastid where they are incorporated by homologous recombination. This plastid transformation procedure is also suitable for practicing the disclosed compositions and methods.

### A. ACBP2

ACBP2 genes useful in the vectors described herein include naturally occurring ACBP2. Naturally occurring ACBP2 is known in the art. An ACMP2 sequence is found in the GenBank/EMBL data library under accession numbers NM_118916 (ACBP2).

Other genes useful for conferring drought resistance to plants include variants of ACPB2. In some embodiments, the variant is a synthetic nucleic acid. Preferably, the variants include less than 25, less than 20, less than 15, less than 10, less than 5, less than 4, less than 3, or less than 2 amino acid substitutions, rearrangements, insertions, and/or deletions relative to Arabidopsis ACBP2. In this regard, the term "variant" can encompass fragments, derivatives, and homologs of Arabidopsis ACBP2. The ACBP2 homolog is preferably an ACBP2-like sequence with at least 77% DNA homology to ACBP2, that can be expressed in guard cells from its own promoter in the wild type and when overexpressed in transgenic plants, like ACBP2, it downregulates *HAB1* and upregulates *AtrbohD* and *AtrbohF.* More preferably, the variants include peptide sequences having at least 90% amino acid sequence identity to Arabidopsis ACBP2.

Sequence similarity can be determined using methods known in the art. For example, determine sequence identity, sequences can be aligned using the methods and computer programs, including BLAST, available over the world wide web at ncbi.nlm.nih.gov/BLAST. See, e.g., Altschul, et al. J. Mol. Biol. 215:403-410 (1990). Another alignment algorithm is FASTA, available in the Genetics Computing Group (GCG) package, from Madison, Wis., USA, a wholly owned subsidiary of Oxford Molecular Group, Inc. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, Calif., USA. Of particular interest are alignment programs that permit gaps in the sequence. The Smith-Waterman is one type of algorithm that permits gaps in sequence alignments. Meth. Mol. Biol., 70: 173-187 (1997). Also, the GAP program using the Needleman and Wunsch alignment method can be utilized to align sequences. J. Mol. Biol., 48: 443-453 (1970).

The variant of ACBP2 is a mutant, isolated from a host cell as described herein. and is encoded by a nucleic acid that hybridizes under stringent conditions to a nucleic acid encoding an Arabidopsis ACBP2 or another known ACBP2 according to SEQ ID NO: 6 or peptide sequences having at least 90% amino acid identity to SEQ ID NO: 6 and having Arabidopsis thaliana acyl-Coenzme A-binding protein 2 activity.

### B. Promoters

The selection of the promoter used in expression vectors determines the spatial and temporal expression pattern of the transgene in the transgenic plant. Promoters vary in their strength, i.e., ability to promote transcription. Selected promoters express transgenes in specific cell types (such as leaf epidermal cells, mesophyll cells, root cortex cells) or in specific tissues or organs (roots, leaves or flowers, for example) and the selection reflects the desired location of accumulation of the gene product. Alternatively, the selected promoter drives expression of the gene under various inducing conditions.

### 1. Constitutive Promoters

Suitable constitutive promoters for nuclear-encoded expression include, for example, the core promoter of the Rsyn7 promoter and other constitutive promoters disclosed in U.S. Pat. No. 6,072,050; the core CAMV 35S promoter, (Odell, et al., Nature 313:810-812 (1985)); rice actin (McElroy, et al., Plant Cell 2:163-171 (1990),); ubiquitin (Christensen, et al., Plant Mol. Biol., 12:619-632 (1989) and Christensen, et al., Plant Mol. Biol. 18:675-689 (1992)); pEMU (Last, et al., Theor. Appl. Genet. 81:581-588 (1991)); MAS (Velten, et al., EMBO J., 3:2723-2730 (1984)); and ALS promoter (U.S. Pat. No. 5,659,026). Other constitutive promoters include, for example, U.S. Pat. Nos. 5,608,149; 5,608,144; 5,604,121; 5,569,597; 5,466,785; 5,399,680; 5,268,463; 5,608,142.

### 2. Tissue Specific Promoters

"Tissue-preferred" promoters can be used to target a gene expression within a particular tissue such as seed, leaf or root tissue. Tissue-preferred promoters are described in Yamamoto, et al., Plant J. 12(2)255-265 (1997); Kawamata, et al., Plant Cell Physiol. 38(7):792-803 (1997); Hansen, et al., Mol. Gen. Genet. 254(3):337-343 (1997); Russell, et al., Transgenic Res. 6(2):157-168 (1997); Rinehart, et al., Plant Physiol. 112(3):1331-1341 (1996); Van Camp, et al., Plant Physiol. 112(2):525-535 (1996); Canevascini, et al., Plant Physiol. 112(2):513-524 (1996); Yamamoto, et al., Plant Cell Physiol. 35(5):773-778 (1994); Lam, Results Probl. Cell Differ. 20:181-196 (1994); Orozco, et al., Plant Mol. Biol. 23(6):1129-1138 (1993); Matsuoka, et al., Proc Natl. Acad. Sci. USA 90(20):9586-9590 (1993); and Guevara-Garcia, et al., Plant J. 4(3):495-505 (1993). Suitable tissue specific expression patterns include green tissue specific, root specific, stem specific, and flower specific.

Promoters suitable for expression in green tissue include many which regulate genes involved in photosynthesis, and many of these have been cloned from both monocotyledons and dicotyledons. Leaf-specific promoters are known in the art. See, for example, Yamamoto, et al., Plant J. 12(2):255-265 (1997); Kwon, et al., Plant Physiol. 105:357-67 (1994); Yamamoto, et al. Plant Cell Physiol. 35(5):773-778 (1994); Gotor, et al. Plant J. 3:509-18 (1993); Orozco, et al., Plant Mol. Biol. 23(6):1129-1138 (1993); and Matsuoka, et al. Proc. Natl. Acad. Sci. USA 90(20):9586-9590 (1993). Another example is the maize PEPC promoter from the phosphoenol carboxylase gene (Hudspeth & Grula, Plant Molec.Biol. 12: 579-589 (1989)), and promoters include those encoding rbsC (Coruzzi et al., EMBO J., 3:1671-1697 (1984)).

Root-preferred promoters are known and may be selected from the many available from the literature or isolated de novo from various compatible species. See, for example, Hire et al. Plant Mol. Biol. 20(2): 207-218 (1992)(soybean root-specific glutamine synthetase gene); Keller and Baumgartner, Plant Cell, 3(10):1051-1061 (1991) (root-specific control element in the GRP 1.8 gene of French bean); Sanger et al., Plant Mol. Biol. 14(3):433-443 (1990) (root-specific promoter of the mannopine synthase (MAS) gene of Agrobacterium tumefaciens); and Miao et al., Plant Cell, 3(1):1 1'-22 (1991) (full-length cDNA clone encoding cytosolic glutamine synthetase (GS), which is expressed in roots and root nodules of soybean). See also U.S. Patent Nos. 5,837,876; 5,750,386; 5,633,363; 5,459,252; 5,401,836; 5,110,732; 5,023,179 and 7,285,656. A suitable promoter for root specific expression is that described by de Framond FEBS 290: 103-106 (1991); EP 0 452 269 to de Framond and a root-specific promoter is that from the T-1 gene. SAHH or SHMT (Sivanandan et al., Biochimica et Biophysica Acta, 1731:202-208, 2005) is specific for root-specific expression. Also, the Cauliflower Mosaic Virus (CaMV) 35S promoter has been reported to have root-specific and leaf-specific modules in its promoter region (Benfey et al., EMBO J., 8:2195-2202, 1989). Other tissue-specific promoters are well known and widely available to those of ordinary skill in the art.

A suitable stem specific promoter is that described in U.S. Pat. No. 5,625,136 and which drives expression of the maize trpA gene.

Plastid specific promoters include the PrbcL promoter [Allison, et al., EMBO J. 15:2802-2809 (1996); Shiina, et al., Plant Cell, 10: 1713-1722 (1998)]; the PpsbA promoter [Agrawal, et al., Nucleic Acids Research, 29: 1835-1843 (2001)]; the Prrn 16 promoter [Svab & Maliga, Proc. Natl. Acad. Sci. USA 90: 913-917 (1993), Allison, et al., EMBO 15: 2802-2809 (1996)]; the PaccD promoter (WO97/06250; Hajdukiewicz, et al., EMBO J. 16: 4041-4048 (1997)).

### 3. Inducible Promoters

Inducible promoters, for example, chemical-regulated promoters can be used to modulate the expression of a gene in a plant through the application of an exogenous chemical regulator. Depending upon the objective, the promoter may be a chemical-inducible promoter, where application of the chemical induces gene expression, or a chemical-repressible promoter, where application of the chemical represses gene expression. Further, a wide variety of inducible promoters are also well known and widely available to those of ordinary skill in the art. Inducible promoter systems used successfully in plants have been extensively reviewed (Padidam, Curr. Opin. Plant Biol. 6:169 (2003); Wang, et al. Trans. Res.:12, 529 (2003); Gatz and Lenk, Trends Plant Sci. 3:352 (1998)). These inducible systems may be activated by chemicals such as tetracycline, pristamycin, pathogen, light, glucocorticoid, estrogen, copper, herbicide safener, ethanol, IPTG (iso-propyl β-D-1-thiogalactopyranoside), and pathogens.

Useful Chemical-inducible promoters and include, but are not limited to, the maize 1n2-2 promoter, which is activated by benzenesulfonamide herbicide safeners, the maize GST promoter, which is activated by hydrophobic electrophilic compounds that are used as pre-emergent herbicides, and the tobacco PR-1 a promoter, which is activated by salicylic acid. Other chemical-regulated promoters of interest include steroid-responsive promoters (see, for example, the glucocorticoid-inducible promoter in Schena, et al. Proc. Natl. Acad. Sci. USA, 88:10421-10425 (1991) and McNellis, et al. Plant J., 14(2):247-257(1998)) and tetracycline-inducible and tetracycline-repressible promoters (see, for example, Gatz, et al., Mol. Gen. Genet. 227:229-237 (1991), and U.S. Patent Nos. 5,814,618 and 5,789,156),

Another suitable category of inducible promoters is that which is wound inducible. Numerous promoters have been described which are expressed at wound sites. Preferred promoters of this kind include those described by Stanford, et al., Mol. Gen. Genet. 215:200-208 (1989), Xu, et al., Plant Molec. Biol., 22: 573-588 (1993), Logemann, et al., Plant Cell, 1: 151-158 (1989), Rohrmeier & Lehle, Plant Molec. Biol., 22: 783-792 (1993), Firek, et al., Plant Molec. Biol., 22: 129-142 (1993), and Warner, et al., Plant J., 3: 191-201 (1993).

### C. Transcriptional Terminators

A variety of transcriptional terminators are available for use in expression cassettes. These are responsible for the termination of transcription beyond the transgene and its correct polyadenylation. Accordingly, at the extreme 3' end of the transcript of the transgene, a polyadenylation signal can be engineered. A polyadenylation signal refers to any sequence that can result in polyadenylation of the mRNA in the nucleus prior to export of the mRNA to the cytosol, such as the 3' region of nopaline synthase (Bevan, et al. Nucleic Acids Res., 11:369-385 (1983). Other transcriptional terminators are those that are known to function in plants and include the CaMV 35S terminator, the tm1 terminator, the nopaline synthase terminator and the pea rbcS E9 terminator. These are used in both monocotyledonous and dicotyledonous plants.

### D. Sequences for the Enhancement or Regulation of Expression

Numerous sequences have been found to enhance gene expression from within the transcriptional unit and these sequences can be used in conjunction with the genes to increase their expression in transgenic plants. For example, various intron sequences such as introns of the maize Adh1 gene have been shown to enhance expression, particularly in monocotyledonous cells. In addition, a number of non-translated leader sequences derived from viruses are also known to enhance expression, and these are particularly effective in dicotyledonous cells.

### E. Coding Sequence Optimization

The coding sequence of the selected gene may be genetically engineered by altering the coding sequence for increased or optimal expression in the crop species of interest. Methods for modifying coding sequences to achieve optimal expression in a particular crop species are well known (see, e.g. Perlak, et al., Proc. Natl. Acad. Sci. USA, 88: 3324 (1991); and Koziel ,et al, Biotechnol. 11: 194 (1993)).

### F. Targeting Sequences

The disclosed vectors may further include, within the region that encodes the protein to be expressed, one or more nucleotide sequences encoding a targeting sequence. A "targeting" sequence is a nucleotide sequence that encodes an amino acid sequence or motif that directs the encoded protein to a particular cellular compartment, resulting in localization or compartmentalization of the protein. Presence of a targeting amino acid sequence in a protein typically results in translocation of all or part of the targeted protein across an organelle membrane and into the organelle interior. Alternatively, the targeting peptide may direct the targeted protein to remain embedded in the organelle membrane. The "targeting" sequence or region of a targeted protein may contain a string of contiguous amino acids or a group of noncontiguous amino acids. The targeting sequence can be selected to direct the targeted protein to a plant organelle such as a nucleus, a microbody (e.g., a peroxisome, or a specialized version thereof, such as a glyoxysome) an endoplasmic reticulum, an endosome, a vacuole, a plasma membrane, a cell wall, a mitochondria, a chloroplast or a plastid.

A chloroplast targeting sequence is any peptide sequence that can target a protein to the chloroplasts or plastids, such as the transit peptide of the small subunit of the alfalfa ribulose-biphosphate carboxylase (Khoudi, et al., Gene, 197:343-351 (1997)).

A peroxisomal targeting sequence refers to any peptide sequence, either N-terminal, internal, or C-terminal, that can target a protein to the peroxisomes, such as the plant C-terminal targeting tripeptide SKL (Banjoko & Trelease, ,Plant Physiol., 107:1201-1208 (1995); Wallace, et al., "Plant Organellular Targeting Sequences," in Plant Molecular Biology, Ed. R. Croy, BIOS Scientific Publishers Limited (1993) pp. 287-288, and peroxisomal targeting in plant is shown in , Volokita, The Plant J., 361-366 (1991)).

Plastid targeting sequences are known in the art and include the chloroplast small subunit of ribulose-1,5-bisphosphate carboxylase (Rubisco) (de Castro Silva Filho et al. Plant Mol. Biol. 30:769-780 (1996); Schnell et al. J. Biol. Chem. 266(5):3335-3342 (1991)); 5-(enolpyruvyl)shikimate-3-phosphate synthase (EPSPS) (Archer, et al., J. Bioenerg. Biomemb., 22(6):789-810 (1990)); tryptophan synthase (Zhao et al.,, J. Biol. Chem., 270(11):6081-6087 (1995)); plastocyanin (Lawrence, et al., J. Biol. Chem., 272(33):20357-20363 (1997)); chorismate synthase (Schmidt, et al., J. Biol. Chem., 268(36):27447-27457 (1993)); and the light harvesting chlorophyll a/b binding protein (LHBP) (Lamppa, et al., J. Biol. Chem., 263:14996-14999 (1988)). *See also* Von Heijne, et al., Plant Mol. Biol. Rep., 9:104-126 (1991); Clark, et al., J. Biol. Chem. 264:17544-17550 (1989); Della-Cioppa et al. Plant Physiol. 84:965-968 (1987); Romer et al. Biochem. Biophys. Res. Commun. 196:1414-1421 (1993); and Shah et al. Science, 233:478-481 (1986). Alternative plastid targeting signals have also been described in the following: US 2008/0263728; Miras, et al. J Biol Chem, 277(49) (2002): 47770-8 (2002); Miras, et al., J Biol Chem, 282: 29482-29492(2007).

### G. Selectable Markers

The expression cassettes described herein may encode a selectable marker to enable selection of transformation events. There are many methods that have been described for the selection of transformed plants [for review see (Miki, et al., Journal of Biotechnology, 107:193-232 (2004)) and references incorporated within]. Selectable marker genes that have been used extensively in plants include the neomycin phosphotransferase gene nptII (U.S. Patent Nos. 5,034,322, U.S. 5,530,196), hygromycin resistance gene (U.S. Patent No. 5,668,298), the bar gene encoding resistance to phosphinothricin (U.S. Patent No. 5,276,268), the expression of aminoglycoside 3"-adenyltransferase (aadA) to confer spectinomycin resistance (U.S. Patent No. 5,073,675), the use of inhibition resistant 5-enolpyruvyl-3-phosphoshikimate synthetase (U.S. Patent No. 4,535,060) and methods for producing glyphosate tolerant plants (U.S. Patent No. 5,463,175; U.S. Patent No. 7,045,684). Methods of plant selection that do not use antibiotics or herbicides as a selective agent have been previously described and include expression of glucosamine-6-phosphate deaminase to inactive glucosamine in plant selection medium (U.S. Pat. No. 6,444,878), and a positive/negative system that utilizes D-amino acids (Erikson, et al., Nat Biotechnol, 22:455-8 (2004)). European Patent Publication No. EP 0 530 129 describes a positive selection system which enables the transformed plants to outgrow the non-transformed lines by expressing a transgene encoding an enzyme that activates an inactive compound added to the growth media. U.S. Patent No. 5,767,378 describes the use of mannose or xylose for the positive selection of transgenic plants. Methods for positive selection using sorbitol dehydrogenase to convert sorbitol to fructose for plant growth have also been described (WO 2010/102293). Screenable marker genes include the beta-glucuronidase gene (Jefferson, et al., EMBO J., 6:3901-3907 (1987); U.S. Patent No. 5,268,463) and native or modified green fluorescent protein gene (Cubitt, et al., Trends Biochem. Sci. 20: 448-455 (1995); Pan, et al., Plant Physiol., 112: 893-900 (996).

Transformation events can also be selected through visualization of fluorescent proteins such as the fluorescent proteins from the nonbioluminescent Anthozoa species which include DsRed, a red fluorescent protein from the Discosoma genus of coral (Matz, et al., Nat Biotechnol, 17:969-73 (1999)). An improved version of the DsRed protein has been developed (Bevis and Glick, Nat Biotech, 20:83-87 (2002)) for reducing aggregation of the protein. Visual selection can also be performed with the yellow fluorescent proteins (YFP) including the variant with accelerated maturation of the signal (Nagai, et al., Nat Biotech., 20:87-90 (2002)), the blue fluorescent protein, the cyan fluorescent protein, and the green fluorescent protein (Sheen, et al., Plant J, 8:777-84 (1995); Davis and Vierstra, Plant Molecular Biology, 36:521-528 (1998)). A summary of fluorescent proteins can be found in Tzfira et al. (Tzfira, et al., Plant Molecular Biology, 57:503-516 (2005)) and Verkhusha and Lukyanov Nat Biotech, 22:289-296 (2004)) Improved versions of many of the fluorescent proteins have been made for various applications. Use of the improved versions of these proteins or the use of combinations of these proteins for selection of transformants will be obvious to those skilled in the art. It is also practical to simply analyze progeny from transformation events for the presence of the PHB thereby avoiding the use of any selectable marker.

For plastid transformation constructs, a preferred selectable marker is the spectinomycin-resistant allele of the plastid 16S ribosomal RNA gene (Staub and Maliga, Plant Cell, 4:39-45 (1992); Svab, et al., Proc. Natl. Acad. Sci. USA, 87: 8526-8530 (1990)). Selectable markers that have since been successfully used in plastid transformation include the bacterial aadA gene that encodes aminoglycoside 3'-adenyltransferase (AadA) conferring spectinomycin and streptomycin resistance (Svab, et al., Proc. Natl. Acad. Sci. USA, 90:913-917 (1993)), nptII that encodes aminoglycoside phosphotransferase for selection on kanamycin (Carrer, et al., Mol. Gen. Genet., 241:49-56 (1993); Lutz, et al., Plant J., 37: 906-913 (2004); Lutz, et al., Plant Physiol., 145:1201-1210 (2007)), aphA6, another aminoglycoside phosphotransferase (Huang, et al, Mol. Genet. Genomics, 268: 19-27 (2002)), and chloramphenicol acetyltransferase (Li, et al. Plant Mol Biol, 76(5-6):443-451 (2010)). Another selection scheme has been reported that uses a chimeric betaine aldehyde dehydrogenase gene (BADH) capable of converting toxic betaine aldehyde to nontoxic glycine betaine (Daniell, et al., Curr. Genet., 39: 109-116 (2001)).

### III. PLANTS/PLANT MATERIAL

A wide variety of plants and plant cell systems can be engineered to express an ACBP2 polypeptide or a functional fragment or variant of ACBP2. Plant material such as leaves, stems, roots, flowers or flower parts, fruits, pollen, egg cells, zygotes, seeds, cuttings, cell or tissue cultures, or any other part or product of a plant can thus be obtained, thus genetically modified show improved drought tolerance.

The genetically modified plant or plant material comprises one or more genes encoding an ACBP2 polypeptide or a functional fragment or variant of ACBP2. In some embodiments the genetically modified plant/plant material comprises two nucleotide sequences encoding the two or more ACBP2s, which may each be contained on separate expression vectors, or, on single expression vector under the control of a common promoter..

In preferred embodiments, target plants and plant cells for engineering include monocotyledonous and dicotyledonous plants, such as crops, including grain crops (for example, wheat, maize, rice, millet, barley), tobacco, fruit crops (for example, tomato, strawberry, orange, grapefruit, banana), forage crops (for example, alfalfa), root vegetable crops (for example, carrot, potato, sugar beets, yam), leafy vegetable crops (for example, lettuce, spinach); flowering plants (for example, petunia, rose, chrysanthemum), conifers and pine trees (for example, pine fir, spruce); oil crops (for example, sunflower, rape seed); and plants used for experimental purposes (for example, Arabidopsis). Other examples include plants that are typically grown in groups of more than about 10 plants in order to harvest the entire plant or a part of the plant, for example, a fruit, a flower or a crop, for example, tobacco, grain, that the plants bear, etc.), trees (i.e., fruit trees, trees grown for wood production, trees grown for decoration, etc.), flowers of any kind (i.e., plants grown for purposes of decoration, for example, following their harvest), cactuses. Further examples of plants in which the ACBP2s may be expressed include Viridiplantae, Streptophyta, Embryophyta, Tracheophyta, Euphyllophytes, Spermatophyta, Magnoliophyta, Liliopsida, Commelinidae, Poales, Poaceae, Oryza, Oryza sativa, Zea, Zea mays, Hordeum, Hordeum vulgare, Triticum, Triticum aestivum, Eudicotyledons, Core eudicots, Asteridae, Euasterids, Rosidae, Eurosids II, Brassicales, Brassicaceae, Arabidopsis, Magnoliopsida, Solananae, Solanales, Solanaceae, Solanum, and Nicotiana. Additional plants that can be transformed using the vectors described herein include, but not limited to, species from the genera Anacardium, Arachis, Asparagus, Atropa, Avena, Brassica, Citrus, Citrullus, Capsicum, Carthamus, Cocos, Coffea, Cucumis, Cucurbita, Daucus, Elaeis, Fragaria, Glycine, Gossypium, Helianthus, Heterocallis, Hordeum, Hyoscyamus, Lactuca, Linum, Lolium, Lupinus, Lycopersicon, Malus, Manihot, Majorana, Medicago, Nicotiana, Olea, Oryza, Panieum, Panneserum, Persea, Phaseolus, Pistachia, Pisum, Pyrus, Prunus, Raphanus, Ricinus, Secale, Senecio, Sinapis, Solanum, Sorghum, Theobromus, Trigonella, Titicum, Vicia, Vitis, Vigna, and Zea.

### IV. METHOD FOR PRODUCING DROUGHT RESISTANT PLANT/PLANT CELLS

The plants and plant cells/material described herein may be obtained by engineering one or more of the vectors expressing an ACBP2 polypeptide or a functional fragment or variant of ACBP2 as described herein into a variety of plant cell types, including but not limited to, protoplasts, tissue culture cells, tissue and organ explants, pollens, embryos, as well as whole plants.

Transformation protocols as well as protocols for introducing nucleotide sequences into plants may vary depending on the type of plant or plant cell targeted for transformation. Suitable methods of introducing nucleotide sequences into plant cells and subsequent insertion into the plant genome include microinjection (Crossway, et al., Biotechniques, 4:320-334 (1986)), electroporation (Riggs, et al., Proc. Natl. Acad. Sci. USA, 83:5602-5606 (1986)), Agrobacterium-mediated transformation (Townsend, et al., U.S. Pat. No. 5,563,055; Horsch, et al., Science, 227: 1227-1231 (1985)), direct gene transfer (Paszkowski, et al. EMBO J., 3:2717-2722 (1984)), and ballistic particle acceleration (see, for example, Sanford et al., U.S. Pat. No. 4,945,050;Tomes, et al., Plant Cell, Tissue, and Organ Culture: Fundamental Methods, ed. Gamborg and Phillips (Springer-Verlag, Berlin) (1995); and McCabe, et al., Biotechnology 6:923-926 (1988)). Also see Weissinger, et al. Ann. Rev. Genet., 22:421-477 (1988); Sanford, et al., Particulate Science and Technology, 5:27-37 (1987) (onion); Christou, et al., Plant Physiol., 87:671-674 (1988) (soybean); McCabe, et al., BioTechnology, 6:923-926 (1988) (soybean); Finer and McMullen, In Vitro Cell Dev. Biol., 27P:175-182 (1991) (soybean); Singh, et al., Theor. Appl. Genet., 96:319-324 (1998)(soybean); Dafta, et al., Biotechnology, 8:736-740 (1990) (rice); Klein, et al., Proc. Natl. Acad. Sci. USA, 85:4305-4309 (1988) (maize); Klein, et al., Biotechnology, 6:559-563 (1988) (maize); Tomes, U.S. Pat. No. 5,240,855; Buising, et al., U.S. Pat. Nos. 5,322,783 and 5,324,646; Tomes, et al. (1995) in Plant Cell, Tissue, and Organ Culture: Fundamental Methods, ed. Gamborg (Springer-Verlag, Berlin) (maize); Klein, et al., Plant Physiol., 91:440-444 (1988) (maize); Fromm, et al., Biotechnology, 8:833-839 (1990) (maize); Hooykaas-Van Slogteren, et al., Nature, 311:763-764 (1984); Bowen, et al., U.S. Pat. No. 5,736,369 (cereals); Bytebier, et al., Proc. Natl. Acad. Sci. USA, 84:5345-5349 (1987) (Liliaceae); De Wet, et al. in The Experimental Manipulation of Ovule Tissues, ed. Chapman et al. (Longman, N.Y.), pp. 197-209 (1985) (pollen); Kaeppler et al. Plant Cell Reports 9:415-418 (1990) and Kaeppler, et al., Theor. Appl. Genet., 84:560-566 (1992) (whisker-mediated transformation); D'Halluin, et al., Plant Cell,, 4:1495-1505 (1992) (electroporation); Li, et al., Plant Cell Reports, 12:250-255 (1993); Christou and Ford, Annals of Botany, 75:407-413 (1995) (rice); Osjoda, et al., Nature Biotechnology, 14:745-750 (1996) (maize via Agrobacterium tumefaciens);

Methods for protoplast transformation and/or gene gun for Agrisoma technology are described in WO 2010/037209. Methods for transforming plant protoplasts are available including transformation using polyethylene glycol (PEG), electroporation, and calcium phosphate precipitation (see for example Potrykus, et al., Mol. Gen. Genet., 199:183-188 (1985); Potrykus, et al., Plant Molecular Biology Reporter, 3:117-128 (1985). Methods for plant regeneration from protoplasts have also been described [Evans et al., in Handbook of Plant Cell Culture, Vol 1, (Macmillan Publishing Co., New York, 1983); Vasil, IK in Cell Culture and Somatic Cell Genetics (Academic, Orlando, 1984)].

Methods for transformation of plastids such as chloroplasts are known in the art. See, for example, Svab, et al., Proc. Natl. Acad. Sci. USA, 87:8526-8530 (1990); Svab and Maliga, Proc. Natl. Acad. Sci. USA, 90:913-917 (1993); Svab and Maliga, EMBO J. 12:601-606 (1993) and Staub and Maliga, Plant J. 6:547-553 (1994); Kuehnle, US Publication No. 2009/7618819. The method relies on particle gun delivery of DNA containing a selectable marker and targeting of the DNA to the plastid genome through homologous recombination. Additionally, plastid transformation may be accomplished by transactivation of a silent plastid-borne transgene by tissue-preferred expression of a nuclear-encoded and plastid-directed RNA polymerase(McBride, et al., Proc. Natl. Acad. Sci. USA, 91:7301-7305 (1994)) or by use of an integrase, such as the phiC31 phage site-specific integrase, to target the gene insertion to a previously inserted phage attachment site (Lutz, et al., Plant J, 37:906-13 (2004)). Plastid transformation vectors can be designed such that the transgenes are expressed from a promoter sequence that has been inserted with the transgene during the plastid transformation process or, alternatively, from an endogenous plastidial promoter such that an extension of an existing plastidial operon is achieved (Herz, et al., Transgenic Research, 14:969-982 (2005)). An alternative method for plastid transformation as described in WO 2010/061186 wherein RNA produced in the nucleus of a plant cell can be targeted to the plastid genome can also be used. Inducible gene expression from the plastid genome using a synthetic riboswitch has also been reported (Verhounig, et al., Proc Natl Acad Sci U S A,107: 6204-6209 (2010)). Methods for designing plastid transformation vectors are described by Lutz, et al., Plant Physiol, 145:1201-10 (2007).

Recombinase technologies which are useful for producing the disclosed transgenic plants include the cre-lox, FLP/FRT and Gin systems. Methods by which these technologies can be used for the purpose described herein are described for example in U.S. Pat. No. 5,527,695; Dale And Ow, Proc. Natl. Acad. Sci. USA, 88:10558-10562 (1991); Medberry, et al., Nucleic Acids Res. 23:485-490 (1995).

The engineered plant/plant material is selected or screened for transformants (i.e., those that have incorporated or integrated the introduced gene construct(s)) following the approaches and methods described below or screening methods known in the art. Following transformation by any one of the methods described above, procedures that can be used to obtain a transformed plant expressing the transgenes include, but are not limited to: selecting the plant cells that have been transformed on a selective medium; regenerating the plant cells that have been transformed to produce differentiated plants; selecting transformed plants expressing the transgene producing the desired level of desired polypeptide(s) in the desired tissue and cellular location.

A transformed plant cell, callus, tissue, or plant may be identified and isolated by selecting or screening the engineered plant material for traits encoded by the selection marker genes present on the introduced expression cassette. For instance, selection may be performed by growing the engineered plant material on media containing inhibitory amount of the antibiotic or herbicide to which the transforming gene construct confers resistance. Further, transformed plants and plant material may also be identified by screening for the activities of any visible marker genes (e.g., the β-glucuronidase, luciferase, B or C1 genes) that may be present on the vectors described herein. Such selection and screening methodologies are well known to those skilled in the art. Alternatively or in addition, screening may be for improved drought tolerance as taught herein, for example, by observing a reduction in growth-inhibition.

Physical and biochemical methods may also be used to identify plant or plant cell transformants containing the gene constructs/vectors described herein. These methods include, but are not limited to: 1) Southern analysis or PCR amplification for detecting and determining the structure of the recombinant DNA insert; 2) Northern blot, S 1 RNase protection, primer-extension or reverse transcriptase-PCR amplification for detecting and examining RNA transcripts of the gene constructs; 3) enzymatic assays for detecting enzyme activity, where such gene products are encoded by the gene construct; 4) protein gel electrophoresis (PAGE), Western blot techniques, immunoprecipitation, or enzyme-linked immunoassays, where the gene construct products are proteins. Additional techniques, such as in situ hybridization, enzyme staining, and immunostaining, also may be used to detect the presence or expression of the recombinant construct in specific plant organs and tissues. The methods for doing all these assays are well known to those skilled in the art. In a specific embodiment, the selectable marker gene nptII, which specifies kanamycin-resistance, is used in nuclear transformation.

The cells that have been transformed may be grown into plants in accordance with conventional techniques. See, for example, McCormick, et al., Plant Cell Reports 5:81-84(1986). These plants may be grown, and either pollinated with the same transformed variety or different varieties, and the resulting hybrid having constitutive expression of the desired phenotypic characteristic identified. Two or more generations may be grown to ensure that constitutive expression of the desired phenotypic characteristic is stably maintained and inherited and then seeds harvested to ensure constitutive expression of the desired phenotypic characteristic has been achieved. An isolated transformant may be regenerated into a plant and progeny thereof (including the immediate and subsequent generations) via sexual or asexual reproduction or growth. Alternatively, the engineered plant material may be regenerated into a plant before subjecting the derived plant to selection or screening for the marker gene traits. Procedures for regenerating plants from plant cells, tissues or organs, either before or after selecting or screening for marker gene(s), are well known to those skilled in the art.

In plastid transformation procedures, further rounds of regeneration of plants from explants of a transformed plant or tissue can be performed to increase the number of transgenic plastids such that the transformed plant reaches a state of homoplasmy (all plastids contain uniform plastomes containing transgene insert).

### V. METHOD FOR IDENTIFYING GENES WHICH CONFER DROUGHT RESISTANCE

Methods are provided for identifying variants and homologs of ACPB2, which, like ACPB2, confer drought resistance. An exemplary screening method involves introducing an exogenous nucleic acid into a host cell, producing a test cell, where the host cell is one that exhibits growth inhibition in drought conditions when water is restricted or withheld to a growth-inhibiting level for a growth-inhibiting period of time. When an exogenous nucleic acid comprising a nucleotide sequence that encodes an ACPB2 or ACBP2-like polypeptide is introduced into the host cell, growth inhibition of the test cell is relieved. Thus, a reduction in growth inhibition indicates that the exogenous nucleic acid encodes an ACPB2 or ACBP2-like polypeptide, where the encoded polypeptide is produced at a level and/or has an activity that relieves the drought-induced growth inhibition. A reduction in growth inhibition includes an at least about 10%, at least about 20%, at least about 30%, at least about 40%, at least about 50%, at least about 60%, at least about 70%, at least about 80%, at least about 90%, or more, reduction in growth inhibition as compared to a non-genetically-modified host.

To generate a subject genetically modified host cell, one or more nucleic acids including nucleotide sequences encoding one or more ACBP2 polypeptides that convey drought tolerance is introduced stably or transiently into a parent host cell, using established techniques, including, but not limited to, electroporation, calcium phosphate precipitation, DEAE-dextran mediated transfection, liposome-mediated transfection, particle bombardment, Agrobacterium-mediated transformation, and the like. For stable transformation, a nucleic acid will generally further include a selectable marker, for example, any of several well-known selectable markers such as neomycin resistance, ampicillin resistance, tetracycline resistance, chloramphenicol resistance, and kanamycin resistance.

The exogenous nucleic acid is inserted into an expression vector. Expression vectors that are suitable for use in prokaryotic and eukaryotic host cells are known in the art, and any suitable expression vector can be used. Examples among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook et al., MOLECULAR CLONING, A LABORATORY MANUAL (2nd Ed., Cold Spring Harbor Laboratory Press, Cold Spring Harbor, N.Y. (1989)).

Where a parent host cell has been genetically modified to produce two or more ACBP2s, nucleotide sequences encoding the two or more ACBP2s will in some embodiments each be contained on separate expression vectors. Where the host cell is genetically modified to express one or more ACBP2s, nucleotide sequences encoding the one or more ACBP2s will in some embodiments be contained in a single expression vector. Where nucleotide sequences encoding the one or more ACBP2s are contained in a single expression vector, in some embodiments, the nucleotide sequences will be operably linked to a common control element (for example, a promoter), such that the common control element controls expression of all of the ACBP2-encoding nucleotide sequences on the single expression vector.

An exogenous nucleic acid will in some embodiments be isolated from a cell or an organism in its natural environment. Methods of isolating the exogenous nucleic acid from test cell are well known in the art. Suitable methods include, but are not limited to, any of a number of alkaline lysis methods that are standard in the art. In other embodiments, the nucleic acid of the cell or organism will be mutated before nucleic acid is isolated from the cell or organism. In other embodiments, the exogenous nucleic acid is synthesized in a cell-free system in vitro.

In some embodiments, the screening method includes further characterizing a candidate gene product. In these embodiments, the exogenous nucleic acid comprising nucleotide sequence(s) encoding an ACBP2(s) are isolated from a test cell as described above. The isolated nucleic acid may be subjected to nucleotide sequence analysis, and the amino acid sequence of the gene product deduced from the nucleotide sequence. In some embodiments, the amino acid sequence of the gene product is compared with other amino acid sequences in a public database of amino acid sequences, to determine whether any significant amino acid sequence identity to an amino acid sequence of a known protein exists.

After the exogenous gene has been identified as having the ability to confer drought resistance, this newly identified ACBP2 variant/homolog can be used to provide plants/plant cells with improved drought resistance.

### A. Exogenous Nucleic Acids

Exogenous nucleic acids that are suitable for introducing into a host cell, to produce a test cell, include, but are not limited to, naturally-occurring nucleic acids isolated from a cell. Exogenous nucleic acids to be introduced into a host cell may be identified by hybridization under stringent conditions to a nucleic acid encoding ACBP2. Exogenous sequences which show 77% or more nucleotide sequence homology with ACBP2 can also be introduced into a host cell to form a test cell. An ACBP2-like sequence with at least 77% DNA homology to ACBP2, that is expressed in guard cells from its own promoter in wild type and when overexpressed in transgenic plants or plant cells for example, like ACBP2, downregulates *HAB1* and upregulates *AtrbohD* and *AtrbohF* similar to ACBP2, are identified as ACBP2-like polypeptides, variants or homologs. More preferably, the sequence homology is, 80% or greater, most preferably, 90% or greater.

Naturally-occurring nucleic acids that have been modified (for example, by mutation) before or subsequent to isolation from a cell; synthetic nucleic acids, e.g., nucleic acids synthesized in a laboratory using standard methods of chemical synthesis of nucleic acids, or generated by recombinant methods; synthetic or naturally-occurring nucleic acids that have been amplified in vitro, either within a cell or in a cell-free system; and the like. Exemplary exogenous nucleic acids include, but are not limited to, genomic DNA; RNA; a complementary DNA (cDNA) copy of mRNA isolated from a cell; recombinant DNA; and DNA synthesized in vitro, e.g., using standard cell-free in vitro methods for DNA synthesis. In some embodiments, exogenous nucleic acids are a cDNA library made from cells, either prokaryotic cells or eukaryotic cells. In some embodiments, exogenous nucleic acids are a genomic DNA library made from cells, either prokaryotic cells or eukaryotic cells.

In some embodiments, for example, where the exogenous nucleic acid is a plurality of exogenous nucleic acids (such as, for example, a cDNA library, a genomic library, or a population of nucleic acids, each encoding an ACBP2 or ACBP2-like polypeptide with a different amino acid sequence, etc.), the exogenous nucleic acids are introduced into a plurality of host cells, forming a plurality of test cells. The test cells are in some embodiments grown in culture under drought conditions such that native cells of the same type would exhibit growth inhibition and/or death; those test cells comprising an exogenous nucleic acid that comprises nucleotide sequences encoding an ACBP2/ACBP2-like polypeptide will grow faster than test cells that do not comprise an exogenous nucleic acid that comprises nucleotide sequences encoding an ACBP2/ACBP2-like polypeptide, or those test cells comprising an exogenous nucleic acid that comprises nucleotide sequences encoding an ACBP2/ACBP2-like polypeptide will live, while test cells that do not comprise an exogenous nucleic acid that comprises nucleotide sequences encoding ACBP2/ACBP2-like polypeptide will die or otherwise be adversely affected.

In other embodiments, the exogenous nucleic acid is a synthetic nucleic acid which comprises for example, a nucleotide sequence encoding a variant ACBP2, for example, an ACBP2 that differs in amino acid sequence by one or more amino acids from a naturally-occurring Arabidopsis ACBP2 or other parent ACBP2. In some embodiments, a variant ACBP2 differs in amino acid sequence by one amino acid, two amino acids, three amino acids, four amino acids, five amino acids, six amino acids, seven amino acids, eight amino acids, nine amino acids, or ten amino acids, or more, compared to the amino acid sequence of a naturally-occurring parent ACBP. In some embodiments, a variant ACBP differs in amino acid sequence by from about 10 amino acids to about 15 amino acids, from about 15 amino acids to about 20 amino acids, from about 20 amino acids to about 25 amino acids, from about 25 amino acids to about 30 amino acids, from about 30 amino acids to about 35 amino acids, from about 35 amino acids to about 40 amino acids, from about 40 amino acids to about 50 amino acids, or from about 50 amino acids to about 60 amino acids, compared to the amino acid sequence of a naturally-occurring parent ACBP.

Manual chemical synthesis of DNA may be accomplished using well-established procedures, or automated chemical synthesis can be performed using one of a number of commercially available machines. The nucleotide sequence of the nucleic acids can be modified for optimal expression based on optimization of nucleotide sequence to reflect the codon bias of the host cell. The skilled artisan appreciates the likelihood of successful expression if codon usage is biased towards those codons favored by the host. Determination of preferred codons can be based on a survey of genes derived from the host cell where sequence information is available. Fragments of full-length proteins can be produced by techniques well known in the art, such as by creating synthetic nucleic acids encoding the desired portions; or by use of Bal 31 exonuclease to generate fragments of a longer nucleic acid.

In still other embodiments, a variant ACBP2 is encoded by a nucleic acid that hybridizes under stringent conditions to a nucleic acid encoding an Arabidopsis ACBP2 or another known ACBP2.

Nucleic acids will in some embodiments be mutated before being introduced into a host cell to form the test cell. In these embodiments, a nucleic acid comprising a nucleotide sequence encoding a naturally-occurring ACBP is mutated, using any of a variety of well-established methods, giving rise to a nucleic acid comprising a nucleotide sequence encoding a variant ACBP2. Nucleotide sequences encoding ACBPs are known in the art, and any known ACBP2-encoding nucleotide sequence can be altered to generate a synthetic nucleic acid for use in a subject method.

Methods of mutating a nucleic acid are well known in the art and include well-established chemical mutation methods, radiation-induced mutagenesis, and methods of mutating a nucleic acid during synthesis. Chemical methods of mutating DNA include exposure of DNA to a chemical mutagen, e.g., ethyl methanesulfonate (EMS), methyl methanesulfonate (MMS), N-nitrosourea (ENU), N-methyl-N-nitro-N'-nitrosoguanidine, 4-nitroquinoline N-oxide, diethylsulfate, benzopyrene, cyclophosphamide, bleomycin, triethylmelamine, acrylamide monomer, nitrogen mustard, vincristine, diepoxyalkanes (for example, diepoxybutane), ICR-170, formaldehyde, procarbazine hydrochloride, ethylene oxide, dimethylnitrosamine, 7,12 dimethylbenz(a)anthracene, chlorambucil, hexamethylphosphoramide, bisulfan, and the like. Radiation mutation-inducing agents include ultraviolet radiation, .gamma.-irradiation, X-rays, and fast neutron bombardment. Mutations can also be introduced into a nucleic acid using, e.g., trimethylpsoralen with ultraviolet light. Random or targeted insertion of a mobile DNA element, e.g., a transposable element, is another suitable method for generating mutations. Mutations can be introduced into a nucleic acid during amplification in a cell-free in vitro system, e.g., using a polymerase chain reaction (PCR) technique such as error-prone PCR. Mutations can be introduced into a nucleic acid in vitro using DNA shuffling techniques (e.g., exon shuffling, domain swapping, and the like). Mutations can also be introduced into a nucleic acid as a result of a deficiency in a DNA repair enzyme in a cell, e.g., the presence in a cell of a mutant gene encoding a mutant DNA repair enzyme is expected to generate a high frequency of mutations (i.e., about 1 mutation/100 genes-1 mutation/10,000 genes) in the genome of the cell. Examples of genes encoding DNA repair enzymes include but are not limited to Mut H, Mut S, Mut L, and Mut U, and the homologs thereof in other species (e.g., MSH 1 6, PMS 1 2, MLH 1, GTBP, ERCC-1, and the like). Methods of mutating nucleic acids are well known in the art, and any known method is suitable for use. See, e.g., Stemple, Nature Reviews, 5:1-7 (2004); Chiang, et al. PCR Methods Appl., 2(3):210-217 (2003); Stemmer, Proc. Natl. Acad. Sci. USA, 91:10747-10751 (1994); and U.S. Pat. Nos. 6,033,861, and 6,773,900.

Thus, for example, a nucleic acid comprising a nucleotide sequence encoding a naturally-occurring ACBP is exposed to a chemical mutagen, as described above, or subjected to radiation mutation, or subjected to an error-prone PCR, and the mutagenized nucleic acid introduced into a genetically modified host cell(s) as described above. Methods for random mutagenesis using a "mutator" strain of bacteria are also well known in the art and can be used to generate a variant ACBP. See, e.g., Greener, et al., Methods in Molecular Biology, 57:375-385 (1995). Saturation mutagenesis techniques employing a polymerase chain reaction (PCR) are also well known and can be used. See, e.g., U.S. Pat. No. 6,171,820. Nucleic acids comprising a nucleotide sequence encoding a variant ACBP are identified by the ability to relieve growth inhibition caused by lead.

### B. Host Cells

The host cell useful in the screening methods described herein can be a eukaryotic cell, a prokaryotic cell, or a cell from a multicellular organism (for example, a cell line).

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); and the like.

### MATERIALS AND METHODS

The materials and methods used in the examples and additional data, are described in Du, et al., Plant, Cell and Environment, July 2012, doi: 10.1111/j.1365-3040.2012.02574.x. [Epub ahead of print],

Plant Materials, Growth Conditions and Stress Treatments

The Arabidopsis mutant acbp2 and ACBP2-OXs (OX-3 and OX-6) were derived from Columbia ecotype Col-0 and Col-6, respectively (Gao, et al., New Phytol., 181:89-102 (2009). Seeds of these Arabidopsis lines were surface sterilized, and sown on MS medium supplemented with 0.8% (w/v) agar and 2% (w/v) sucrose, and grown in a growth chamber under 16-h-light (23°C)/8-h-dark (21°C) cycles. Soil-grown plants were also grown under the similar environmental conditions.

Three-week-old plants potted in soil were used for drought treatment. Water was withheld for 15 d, and then plants were rewatered for 7 d before photography.

### RNA Analysis

TRIzol^{®} reagent was also used for RNA extraction of 12-day-old Arabidopsis seedlings subject to treatment by 100 µM ABA or drought for the various times (0, 1, 3, 6 and 24 h) under continuous white light. First-strand was synthesized using the Superscript First-Strand Synthesis System (Invitrogen). QRT-PCR was performed using StepOne Plus (Applied Biosystems) and FastStart Universal SYBR Green Master (Roche). Transcript changes were analyzed according to Schmittgen and Livak (Nat. Protoc. 3:1101-1108 (2008) from three independent experiments.

Primers used for RT-PCR analysis:
ACBP2, ACBP2-specific primers ML1122, 5'-GTGAGGCGGATTCGCTTGT-3' (SEQ ID NO: 1); and ML1123 5'-TGCGGCGGCGGTAGTC-3' (SEQ ID NO: 2);
HAB1, ML1212 5'-TGCCGTGTCACCTCATT-3' (SEQ ID NO: 11) and ML1213, 5'-TGCGGCGGCGGTAGTC-3' (SEQ ID NO: 12);
AtrbohD-specific primers, ML1220, 5'-ATTACAAGCACCAAACCAG-3' (SEQ ID NO: 13) and ML1221, 5'-TGCCAAGCCATAACATCA-3' (SEQ ID NO: 14);
AtrbohF-specific primers, ML1222, 5'-CTGCGGTTTCGCCATTC-3' (SEQ ID NO: 15) and ML1223, 5'-TGTTTCGTCGGCTCTG-3' (SEQ ID NO: 16).

### ROS Detection in Guard Cells and leaves

Reactive oxygen species (ROS) production in guard cells was analyzed using 2', 7'-dichlorofluorescin diacetate (H₂DCF-DA, Sigma- Aldrich; Lee et al. 1999; Pei et al. 2000). Epidermal peels from 5-week-old plants were incubated in an incubation buffer containing 30 mM KCl and 10 mM Mes-KOH (pH 6.15), under cool white light at 23°C for 2 h. Subsequently, 50 µM H₂DCF-DA was added to the solution and incubated for 15 min. After a brief wash with the incubation buffer, either 50 µM ABA or 0.1% ethanol (control) was added to the solution and treated for 15 min. The epidermal tissues were then washed with distilled water and observed for fluorescence using a Zeiss LSM 510 META microscope, with excitation at 488 nm and emission at 535 nm. DCF fluorescence intensity was measured by LSM Image Browser and Image J (National Institutes of Health).

ROS production in leaves was examined by 3, 3-diaminobenzidine (DAB) staining (Thordal-Christensen et al. 1997). Leaves from 5-week-old plants were harvested and immersed in 1 mg mL-1 DAB solution (pH 3.8) for approximately 8 h at room temperature. Samples were photographed after clearing in 96% boiling ethanol for 10 min.

### Generation of ACBP2-Overexpressing Transgenic Arabidopsis Lines

Transgenic Arabidopsis plants overexpressing ACBP2 were generated by Agrobacterium-mediated transformation (Clough and Bent, Plant J., 16:735-743 (1998) and resultant transformants were subsequently used to test whether ACBP2 overexpression enhances drought tolerance. The ACBP2 full-length cDNA is provided below.

attaaaaagt cttaacgccc acaacgaaaa ggaacttctc tttttgtttg gtgactgaat
cggtggaaaa caacacaagt ttgttccttt ttgtctctcc agatcccttg gcttccttct
tcttcttcat cctcctcgtg agactctgtg ttgaaatttc tctagggttt taattatcgg
cgttggtttt tcgtttttga gaatttgatt tcgtttttag aaataatggg tgattgggct
caacttgctc agtctgtgat cctaggtttg attttctctt accttctcgc taagctgatc
tcgatcgtcg tcacgtttaa agaagacaat ctctctctta ctcgtcaccc agaggagtct
caattggaaa ttaaaccgga aggagttgac tcgcgacgtc tcgattcctc ttgcggtggt
ttcggtggtg aggcggattc gcttgtggcg gagcagggta gctcccgaag tgatagcgtt
gccggtgacg acagtgagga agatgatgat tgggaaggcg tcgagagcac ggaactcgat
gaggctttta gcgccgccac tctttttgtg actaccgccg ccgcagatcg gctttcgcag
aaggtaccga gtgatgtgca gcagcagctt tacggattgt ataagattgc tacggaaggg
ccgtgtactg ctcctcagcc atcagctctc aaaatgactg ctcgtgccaa gtggcaagca
tggcagaaac tgggagctat gccacctgaa gaggcgatgg agaagtatat tgagattgtc
actcagcttt acccaacttg gttagacggt ggcgtgaaag ccggaagtcg gggtggggat
gatgcagcct ccaactcaag aggaaccatg ggaccagttt ttagctcttt ggtttatgat
gaggagtccg aaaatgagtt gaagattgat gccatacacg gatttgctag agaaggagaa
gtcgagaatt tactgaaaag cattgaaagc ggcattcctg taaatgcaag ggacagtgaa
ggtcgcacac cattgcactg ggctatagac cgtggccacc tcaacatcgc caaagttctg
gtcgataaga acgccgatgt gaatgctaag gacaacgaag gccaaacccc tttgcattat
gctgttgtat gcgacagaga agctatcgcc gagtttctgg ttaaacagaa cgcaaacaca
gccgctaaag atgaggatgg aaactctccc cttgatctct gtgaatcaga ctggccctgg
atccgagatt ctgcaaagca ggcagactaa acaaatacta accacgtctt ctctaaatcc
gcaatgtata tgatcaaatg acttagtaag aagcttttct ttactttaaa ctttctttcc
atccctacca catcactggg atgttccaac actatatcac ttggatgtta ccaagtcttg
ttctttgatt tctttcttct tacattttaa caatcttttg ttcctttagg ctttatgata
tgtcgtaacc ggttttgttg gtttgctata aatacacaca tatagaca

(SEQ ID NO: 3)

The ACBP2 full-length cDNA was expressed from the CaMV 35S promoter in binary vector pSMB (Mylne and Botella, Plant Mol. Biol. Rep., 16: 257-262.) for transformation of Arabidopsis (Col-0). Sequence of CaMV 35S promoter-specific forward primer 35SB is 5'-CAATCCCACTATCCTTCGCAAGAC C-3' (SEQ ID NO: 5).

Two independent T2 ACBP2-overexpressing lines (ACBP2-OX3 and ACBP2-OX6) were identified to overexpress the 1.6-kb ACBP2 mRNA in Northern blot analysis (Gao, et al., New Phytol., 181: 89-102 (2009). The amino acid sequence of ACBP2 is provided below.

MGDWAQLAQSVILGLIFSYLLAKLISIVVTFKEDNLSLTRHPEE
SQLEIKPEGVDSRRLDSSCGGFGGEADSLVAEQGSSRSDSVAGDDSEED
DDWEGVESTELDEAFSAATLFVTTAAADRLSQKVPSDVQQQLYGLYKI
ATEGPCTAPQPSALKMTARAKWQAWQKLGAMPPEEAMEKYIEIVTQLY
PTWLDGGVKAGSRGGDDAASNSRGTMGPVFSSLVYDEESENELKIDAIH
GFAREGEVENLLKSIESGIPVNARDSEGRTPLHWAIDRGHLNIAKVLVDK
NADVNAKDNEGQTPLHYAVVCDREAIAEFLVKQNANTAAKDEDGNS
PLDLCESDWPWIRDSAKQAD
(SEQ ID NO: 6).

### Generation of ACBP2pro:GUS Transgenic Arabidopsis Lines

To generate ACBP2pro:GUS fusion constructs, a 1.8-kb fragment of the ACBP2 5'-flanking region was amplified by primer pairs ML805: -1704 to - 1677 in ACBP2 5'-flanking sequence
5'-CTGGATCCAGGAGTCAGCGTCGTATGTC-3'(SEQ ID NO: 7) and ML806: 121-96 in ACBP2 gDNA
5'-TCCCCGGGGTGACGAGTAAGAGAGAG-3' (SEQ ID NO: 8), and cloned into pGEM-T Easy vector (Promega, Madison, WI, USA) to generate plasmid pAT351 (Fig. 4A).

The nucleotide sequence of ACBP2pro:GUS fusion is provided below. 
ttcttttcaaaaagcttcctctggaaacaggagtcagcgtcgtatgtctatcccctggtgttgtcctaacaaatgttgtgagtattagcttcacttcatttcgttacaa
gtgcaggattcctctgttttctcagaaattcattgcgttgaactgcctatttctttccgaaatttacaggccagggatctatccaggattcttcaagctctttacgca
gtgataccttatttcatattttcaccccaagaaggttgtagaagttctctattctcggccacagatcctcagattccagagtactgggaaacactaaaaaacgatg
attggcctgtttgcccattcatctctcaagattgccgccctgcaaatccttccgaagaagcacacaacacagaaactgcacagagagtgtggaaaaagacgt
tagagctggtgggtcttcctctcgatgcagttgagaagctcatagaaggggaaaatatccaatgccggtatggagcacaacacgaatagtctttcaaaattac
cacaggttaagtgacccattacagatcaaagggtaggtaattgagaaaatatctttttttttgtttccttgtattaatctacacgatacagtggggaatgaatcccc
caggcatgtagtttgcttgagaatgtttgattgttggataaaagtcaagctttagctaccattccattgcttttacttacaagtcttgttcaagtatttggttagtgtgtc
ttggagttattaagatgttcagtagtagagtatggtagcctgggttggtgggtagtttcttgtttgagtttagggtattttcgtaatttctttattgtttgtggagtcac
gttgctcatatatagttatagggatatatatcaattagagtcatcatctctcttactctcatggtgacaaagtcacaaaagtgataaactctcctttccactttctaatg
tttcttgcttaaatgagtattccatgtaaaaatgattcactcactcgtatgggtgggttgggttttctcttcatgagatgctcatttgagcaagcaatgtttggaagtg
ggactatttaatatcatgtatgagttttatcttttgttataagaaaaaaagttgggatttcattttcgtgcccacttcacgtgaatctaatgccttcaagtttgtggtcta
ccttattttctcttatggatacatgtcaatttacgtgtcgtatgagtcaagcctttaatcatcccaacaccatgcacgtcttctagatttgttttcgaccacgattccac
cacaaaatttgacattgttattgaattcattaactttttacgtgttattgtgaaatatttatttaattttgttggtaaaaggagcaaaaaagattagggtacaacacgtc
gacttcttccccaattagacccatatgtgatctgtcgtcactcgtcgccaagtttttttatgtgtcgtcttttaaactttgatccaattcattaaaaagtcttaacgccc
acaacgaaaaggaacttctcttttgtttggtgactgaatcggtggaaaacaacacaagtttgttcctttttgtctctccagatcccttggcttccttcttcttcttcat
cctcctcgtgagactctgtgttgaaatttctctagggttttaattatcggcgttggtttttcgtttttgagaatttgatttcgtttttagaaataatgggtgattgggctca
acttgctcagtctgtgatcctaggtttgattttctcttaccttctcgctaagctgatctcgatcgtcgtcacgtttaaagaagacaatctctctcttactcgtcaccccc
gggtacggtcagtcccttatgttacgtcctgtagaaccccaacccgtgaaatcaaaaaactcgacggcctgtgggcattcagtctggatcgcgaaaactgt
ggaattgatcagcgttggtgggaaagcgcgttacaagaaagccgggcaattgctgtgccaggcagttttaacgatcagttcgccgatgcagatattcgtaatt
atgcgggcaacgtctggtatcagcgcgaagtctttataccgaaaggttgggcaggccagcgtatcgtgctgcgtttcgatgcggtcactcattacggcaaag
 
tgtgggtcaataatcaggaagtgatggagcatcagggcggctatacgccatttgaagccgatgtcacgccgtatgttattgccgggaaaagtgtacgtatca
ccgtttgtgtgaacaacgaactgaactggcagactatcccgccgggaatggtgattaccgacgaaaacggcaagaaaaagcagtcttacttccatgatttctt
taactatgccggaatccatcgcagcgtaatgctctacaccacgccgaacacctgggtggacgatatcaccgtggtgacgcatgtcgcgcaagactgtaacc
acgcgtctgttgactggcaggtggtggccaatggtgatgtcagcgttgaactgcgtgatgcggatcaacaggtggttgcaactggacaaggcactagcgg
gactttgcaagtggtgaatccgcacctctggcaaccgggtgaaggttatctctatgaactgtgcgtcacagccaaaagccagacagagtgtgatatctaccc
gcttcgcgtcggcatccggtcagtggcagtgaagggccaacagttcctgattaaccacaaaccgttctactttactggctttggtcgtcatgaagatgcggac
ttacgtggcaaaggattcgataacgtgctgatggtgcacgaccacgcattaatggactggattggggccaactcctaccgtacctcgcattacccttacgctg
aagagatgctcgactgggcagatgaacatggcatcgtggtgattgatgaaactgctgctgtcggctttaacctctctttaggcattggtttcgaagcgggcaa
caagccgaaagaactgtacagcgaagaggcagtcaacggggaaactcagcaagcgcacttacaggcgattaaagagctgatagcgcgtgacaaaaacc
acccaagcgtggtgatgtggagtattgccaacgaaccggatacccgtccgcaagtgcacgggaatatttcgccactggcggaagcaacgcgtaaactcga
cccgacgcgtccgatcacctgcgtcaatgtaatgttctgcgacgctcacaccgataccatcagcgatctctttgatgtgctgtgcctgaaccgttattacggat
ggtatgtccaaagcggcgatttggaaacggcagagaaggtactggaaaaagaacttctggcctggcaggagaaactgcatcagccgattatcatcaccga
atacggcgtggatacgttagccgggctgcactcaatgtacaccgacatgtggagtgaagagtatcagtgtgcatggctggatatgtatcaccgcgtctttgat
cgcgtcagcgccgtcgtcggtgaacaggtatggaatttcgccgattttgcgacctcgcaaggcatattgcgcgttggcggtaacaagaaagggatcttcact
cgcgaccgcaaaccgaagtcggcggcttttctgctgcaaaaacgctggactggcatgaacttcggtgaaaaaccgcagcagggaggcaaacaatga
(SEQ ID NO: 9)
 The amino acid sequence of ACBP2pro:GUS fusion is provided below.

MLRPVETPTREIKKLDGLWAFSLDRENCGIDQRWWESALQESRAIAVPGSFNDQFADADIRNYAG
NVWYQREVFIPKGWAGQRIVLRFDAVTHYGKVWVNNQEVMEHQGGYTPFEADVTPYVIAGKSV
RITVCVNNELNWQTIPPGMVITDENGKKKQSYFHDFFNYAGIHRSVMLYTTPNTWVDDITVVTHV
AQDCNHASVDWQVVANGDVSVELRDADQQVVATGQGTSGTLQVVNPHLWQPGEGYLYELCVT
AKSQTECDIYPLRVGIRSVAVKGQQFLINHKPFYFTGFGRHEDADLRGKGFDNVLMVHDHALMD
WIGANSYRTSHYPYAEEMLDWADEHGIVVIDETAAVGFNLSLGIGFEAGNKPKELYSEEAVNGET
QQAHLQAIKELIARDKNHPSVVMWSIANEPDTRPQVHGNISPLAEATRKLDPTRPITCVNVMFCDA
HTDTISDLFDVLCLNRYYGWYVQSGDLETAEKVLEKELLAWQEKLHQPIIITEYGVDTLAGLHSM
YTDMWSEEYQCAWLDMYHRVFDRVSAVVGEQVWNFADFATSQGILRVGGNKKGIFTRDRKPKS
AAFLLQKRWTGMNFGEKPQQGGKQ
(SEQ ID NO: 10).

Subsequently, a 1.8-kb BamHI-SmaI fragment from pAT351 was cloned into the BamHI-SmaI site of binary vector pBI101.3 to generate pAT353 (Fig. 4B). This construct was verified by DNA sequence analysis and introduced into Arabidopsis (Columbia ecotype) by Agrobacterium tumefaciens-mediated transformation (floral dip approach; Clough and Bent, Plant J. 16: 735-743, 1998).

Histochemical GUS assays were carried out using 5 bromo-4-chloro-3-indolyl-b-D-glucuronide (X-Gluc) according to Liu et al., Plant Cell, 16: 5-20 (2004). Plant samples were immersed and vacuum filtrated in the GUS staining solution for 30 min (100 mM sodium phosphate buffer, pH 7.0, 0.1% Triton X-100, 2 mM potassium ferricyanide, 2 mM potassium ferrocyanide, 1 mg/mL 5 bromo-4-chloro-3-indolyl-β-D-glucuronide), incubated and observed over a period ranging from 3 to 16 h at 37 °C. Subsequently, samples were cleared in 70% ethanol and photographed.

For confocal microscopy, 12-day-old seedlings were incubated in 0.1% Silwet L-77 solution supplemented with 50 mM Imagene Green (Invitrogen, Carlsbad, CA, USA) for 1 h. Samples were then briefly washed in 0.1% Silwet L-77 and photographed under a confocal laser scanning microscope (Zeiss LSM 510 META; Zeiss, Hamburg, Germany).

### ABA Sensitivity at germination

To test ABA sensitivity at germination, over 200 seeds per experiment were sown on MS medium in the presence or absence of 0.75 mM ABA (Sigma-Aldrich, St Louis, MO, USA). After 2 d at 4 °C, seeds were germinated and grown under 16-hour-light (23 °C)/8-hour-dark (21 °C) cycles. Germination was examined daily with a microscope (Leica MZ6; Leica, Solms, Germany), and seedlings were photographed after 7 d. For post-germination growth assays, seeds were germinated on MS medium for 4 d, followed by transfer to MS medium or MS medium supplemented with 100 or 150 mm ABA for 7 d before photography and root measurements.

### Ion leakage measurement

Ion leakage was measured following Welti *et al.* (2002). In brief, detached leaves treated with ABA were agitated in deionized water at 23 °C for 1 h. Conductivity of the solution was measured with a meter (YSI model 55). Subsequently, total ion strength was determined by boiling the solution in a water bath for 10 min and then cooling to 23 °C.

### Sequences

Sequence data included herein can be found in the GenBank/EMBL data libraries under accession numbers NM_118916 (ACBP2), NM_105936 (HAB1), NM_124165 (AtrbohD) and NM_105079 (AtrbohF).

### EXAMPLE 1. ACBP2 Is Induced by ABA and Drought

Abscisic acid (ABA) is a plant hormone that regulates numerous physiological processes, including seed dormancy and germination, seedling establishment, vegetative development and responses to various abiotic stresses, including drought and salinity (Schroeder, Kwak & Allen 2001; Finkelstein, Gampala & Rock 2002; Hirayama & Shinozaki 2007; Fujii & Zhu 2009; Lee & Luan 2012). Both mild and severe water deficiency affects development in plants (Davies & Zhang 1991; Zhu 2002; Skirycz & Inzé 2010; Skirycz et al. 2011). During water-deficit stress, ABA levels rise to regulate the expression of various genes to promote adaptive responses (Christmann et al. 2007; Raghavendra et al. 2010).

To determine the effect of ABA treatment on ACBP2 expression, the expression of ACBP2 was examined by Real-time quantitative PCR (qRT-PCR) using primers ML1122 and ML1123 (SEQ ID NOs: 1 and 2) in 12-day-old seedlings before and after ABA treatment under continuous white light. qRT-PCR was performed using StepOne Plus (Applied Biosystems) and FastStart Universal SYBR Green Master (Roche). Transcript changes were analyzed according to Schmittgen and Livak (Nat. Protoc. 3: 1101-1108 (2008) from three independent experiments.

qRT-PCR results show significant induction of ACBP2 by ABA (100 mM) treatment under continuous white light. Consequently, ACBP2 expression in 12-day-old seedlings was investigated following drought treatment under continuous white light. qRT-PCR results show induction of ACBP2 by drought treatment under continuous white light. ACBP2 was up-regulated at 3, 6 and following 24 h drought treatment in qRT-PCR analyses (Fig. 1B). The induction of ACBP2 by ABA and drought treatments indicates its potential role in drought response.

To further examine *ACBP2* expression in seed germination and early seedling development, histochemical and qRT-PCR analyses were performed.

The data shows that *ACBP2pro:GUS* was expressed in the whole embryo on the first day following germination and was subsequently restricted to the cotyledons and the elongation and differentiation zones of the radicle. From day 4 of germination, *ACBP2pro:GUS* was expressed in the root vascular bundles but not in root tips (data not shown). GUS expression rapidly decreased as germination progressed, whereas ABA treatment suppressed germination and retained GUS expression (data not shown). Similar results on qRT-PCR analyses suggest that *ACBP2* expression can be partially rescued by ABA treatment (Fig. 1C). In conclusion, the expression pattern of *ACBP2* suggests its potential role in seed germination and early seedling development.

### EXAMPLE 2. Drought treatment and stomatal aperture measurement

Drought treatment was performed as described by Osakabe, et al., Journal of Biological Chemistry, 285:9190-9201 (2010). Briefly, 4-week-old plants grown in MS plates were transferred onto filter paper and incubated in a chamber under 40±3% RH for 6 or 8 hours. These plants were then rewatered, and survival rates were calculated after a 3-d recovery. For 3-week-old plants potted in soil, water was withheld for 15 d, and then the plants were rewatered for 7 d before photography. Survival rates are shown in Fig. 2B.

The acbp2 mutant is more sensitive and ACBP2-OXs are more tolerant to drought stress.

Four-week-old ACBP2-OXs plants grown in MS medium and 3-week-old ACBP2-OXs grown in soil were subject to dehydration stress. After drought treatment and recovery, ACBP2-OXs grown in MS medium (Fig. 2A) and soil (Fig. 2B) were both more tolerant to drought stress in comparison to the wild type (Col-0), whereas the acbp2 mutant plants were more sensitive to drought than the wild type (Col-6; Fig. 2A and B).

To further investigate the function of ACBP2 under drought stress, leaf stomatal closure was analyzed in response to ABA in the wild type (Col-0 and Col-6), the acbp2 mutant and ACBP2-OXs.

Stomatal closure experiments were performed according to Pei, et al. Plant Cell, 9:409-423 (1997). Rosette leaves from 4-week-old Arabidopsis plants were incubated for 2 h in stomatal opening solution containing 10 mM KCl, 0.2 mM CaCl₂ and 10 mM Mes-KOH (pH 6.15) under cool white light at 23°C. Subsequently, these leaves were transferred to the same fresh solution supplemented with ABA and incubated for 2 h. Guard cells were photographed under a Leica DMRXA microscope and stomatal aperture was measured with Image J (National Institutes of Health).

Guard cells from ACBP2-OXs showed increased stomatal sensitivity to both 5 µM and 10 µM ABA in comparison to the wild type (Col-0; Fig. 2C). In contrast, guard cells from the acbp2 mutant were less affected than the wild type (Col-6; Fig. 2D). These results suggest a link between ACBP2 and ABA signaling during drought stress by reducing water loss.

*ACBP2-OX* seeds were tested by germination under ABA stress. Germination assays of *ACBP2-OXs (OX-3 and OX-6)* revealed ABA sensitivity.

For seeds germinated on MS medium, no significant difference was noted between transgenic lines and the wild type (Fig. 2E). However, in the presence of exogenous ABA, *ACBP2-OXs* were more sensitive to ABA and germination was slower than the wild type (Col-0; Fig. 2F), whereas significant differences were not detected between the *acbp2* mutant and the wild type (data not shown).

Besides seed germination, ABA is known to regulate root development (Finkelstein *et al.* 2002; Fujii & Zhu 2009). For root growth assays, 4-day-old seedlings germinated on MS medium were transferred to ABA-containing MS medium. After 7 d growth, roots of *ACBP2-OXs* were significantly shorter than the wild type (Col-0; Fig. 2G). Seeds of the *acbp2* mutant were germinated and grown on MS medium for 4 d and were subsequently transferred to fresh MS medium supplemented with 100 or 150 µm ABA for a further 7 d incubation. The root growth of the *acbp2* mutant showed no significant difference in root length to the wild type (Col-6) in the presence of 100 µm ABA. However, the root lengths of *acbp2* mutant seedlings were significantly longer than the wild type (Col-6) with 150 µm ABA (Fig. 2H).

### Example 3. ACBP2-OXs Show Increased ROS Production

Two Arabidopsis guard cell-expressed NAD(P)H oxidases, AtrbohD and AtrbohF, are necessary for ABA-mediated ROS production in guard cells and stomatal closure (Kwak, et al., EMBO J., 22:2623-2633 (2003). From gene expression results, of ACBP2-OXs, AtrbohD and AtrbohF were observed up-regulated by ABA.

ROS production in 5-week-old wild-type and ACBP2-OXs Arabidopsis plants was investigated by 2', 7'-dichlorofluorescin diacetate (H₂DCF-DA) and 3, 3-diaminobenzidine (DAB) staining. H₂DCF-DA is a nonfluorescent compound permeable to the plasma membrane and converted to impermeable dichlorofluorescin (H₂DCF). Cellular peroxidases and H₂O₂ oxidize H₂DCF to generate DCF fluorescence which indicates ROS production.

After incubation with H₂DCF-DA, ROS production in guard cells were recorded at levels similar to the wild-type and ACBP2-OXs Arabidopsis plants without ABA treatment. However, after treatment with 50 µM ABA for 15 min, the guard cells from ACBP2-OXs accumulated more ROS production than the wild-type as indicated by higher fluorescent intensities (Fig. 3A).

The function of *ACBP2* in ABA-associated senescence was tested using detached leaves from the wild type (Col-0 and Col-6), the *acbp2* mutant and *ACBP2-OXs.* After 3 d treatment with 10 mm ABA, leaves from *ACBP2-OXs* displayed greater senescence than the wild type (Col-0), whereas the *acbp2* mutant behaved similar to the wild type (data not shown. The membrane integrity of leaves from the *acbp2* mutant and *ACBP2-OXs* (*OX3* and OX6) was further analysed by ion leakage measurements. The results showed that leakage was higher in *ACBP2-OXs* than the wild type (Col-0; Fig. 3B), consistent with the observation that *ACBP2* overexpression promotes ABA-mediated leaf senescence. In contrast, the difference between the wild type (Col-6) and the *acbp2* mutant was not significant but leaves from *acbp2* mutant plants showed reduced (5.8%) ion leakage after ABA treatment (Fig. 3c).

### Example 4. Spatial Expression of ACBP2

The data shows that ACBP2 is highly expressed in the leaves (5A) and guard cells (Fig 5B). Scale bars = 1 mm (FIG. 5A) and 20 µm (FIG. 5B).

GUS staining also revealed that *ACBP2* was strongly expressed at the early-torpedo, bent cotyledonary and maturation stages. At the maturation stage, *ACBP2* was expressed in most of the embryo except the root apical meristem. Besides expression in embryos, GUS histochemical stains on transgenic Arabidopsis further revealed the pattern of *ACBP2pro:GUS* expression in various organs of Arabidopsis. *ACBP2pro:GUS* was expressed in 3-weekold Arabidopsis seedlings, guard cells of cotyledon and true leaves, and primary and lateral root vascular bundles. In open flowers, *ACBP2pro:GUS* was expressed in the pollen of anther, but not in petals, sepals and pistil. Fluorescent GUS substrate testing confirmed GUS expression in guard cells of seedlings (data not shown, but published in Du, et al., Plant, Cell and Environment, July 2012, doi: 10.1111/j.1365-3040.2012.02574.x. [Epub ahead of print].

### Example 7. Overexpression of ACBP2 Regulates the Expression of AtrbohD, AtrbohF and HAB1.

The role of *ACBP2* in ABA signaling was investigated by using qRT-PCR to determine whether *ACBP2* regulates the genes associated with ABA signalling.

*AtrbohD* and *AtrbohF* encode two NAD(P)H oxidases that generate ROS production in ABA response. Studies have shown that a double mutation in AtrbohD and AtrbohF impaired ABA-induced ROS production and stomatal closure, indicating a principal role for their proteins in ABA signaling (Kwak, et al., EMBO J. 22: 2623-2633 (2003)). In addition, it has been observed that HAB 1 is a dominant negative regulator of ABA signaling and drought tolerance (Saez, et al., Plant J., 37:354-369 (2004).

Thus, expression of *AtrbohD, AtrbohF,* and *HAB1* was compared between the wild type (Col-0) and *ACBP2-OXs.* In addition, expression of ABA-Responsive Element Binding Protein 1 (*AREB1*), ABA Deficient 2 *(ABA2),* 9-cis-Epoxycarotenoid Dioxygenase 3 (*NCED3*), Response to Desiccation 29A (*RD29A*), abscisic acid-insensitive 1 (*ABI1*), phospholipase D *α1 (PLD* α1) and Receptor-like Protein kinase1 (*RPK1*) was compared between the wild type (Col-0) and *ACBP2-OXs.*

Twelve-day-old ACBP2-OXs (OX-3 and OX-6) seedlings grown on Murashige and Skoog (MS) plates were left untreated or treated for 3 h with 100 µM ABA. Gene expression was examined by qRT-PCR.

qRT-PCR analysis showed that the expression of HAB 1 was downregulated in ACBP2-OXs , thereby promoting ABA signaling in ACBP2-OXs and enhance drought tolerance. By contrast, in ABA-treated ACBP2-OXs, the expression of *AtrbohD* and *AtrbohF* were significantly up-regulated consistent with the accumulation of ROS product in guard cells of ACBP2-OXs after ABA treatment. Significant upregulation of AREB1 was observed before and after ABA treatment. *NCED3* expression was downregulated in *ACBP2-OXs* when plants were treated with ABA. There was no significant difference in the expression of Response to Desiccation 29A (*RD29A*), *ABI1, PLDa1* and *Receptor-like Protein kinase1* (*RPK1*) between the wild type (Col-0) and *ACBP2-OXs.* ((Fig. 6A-6I)

### Summary

The Examples demonstrate that ACBP2 mRNA expression is induced by abscisic acid (ABA; a major plant hormone regulating drought tolerance) and drought using quantitative real time-polymerase chain reactions (Fig. 1). The data also shows that transgenic Arabidopsis overexpressing ACBP2 (ACBP2-OXs) are conferred improved drought tolerance. Accumulation of ABA-mediated reactive oxygen species (ROS) production in these cells, promotes stomatal closure, which in turn reduces water loss and improving drought tolerance (Fig. 2A-D and 3). Beta-glucuronidase (GUS) assays verified that ACBP2pro:GUS is expressed in guard cells (Fig. 4 and 5), supporting ACBP2-associated upregulation of ROS production in guard cells. Further investigations revealed that the overexpression of ACBP2 induced the expression of Respiratory Burst Oxidase Homolog D (AtrbohD) and AtrbohF (Fig. 6), two genes encoding two NAD(P)H oxidases that generate ROS production in ABA responses (Kwak, et al., EMBO J., 22:2623-2633 (2003). In addition, ACBP2 overexpression also inhibits the expression of the mRNA encoding the protein Hypersensitive to ABA1 (HAB1), which is a dominant negative regulator of ABA signaling and drought tolerance (Saez, et al., Plant J., 37:354-369 (2004), suggesting a positive role for ACBP2 in these processes. ACBP2 overexpression significantly increased (average of ∼2-fold) the survival rate of transgenic Arabidopsis plants exposed to drought.

Unless defined otherwise, all technical and scientific terms used herein have the same meanings as commonly understood by one of skill in the art to which the disclosed invention belongs.
¡¡¡¡¡¡ACBP2, ACBP2-specific primers ML1122, 5¡⁻ - GTGAGGCGGATTCGCTTGT-3¡⁻ (SEQ. ID No: 1); and ML1123 (SEQ. ID No: 2);
¡¡¡¡¡¡HAB1, ML1212 (.SEQ. ID No: 11) and ML1213, 5¡⁻-TGCGGCGGCGGTAGTC-3¡⁻ (SEQ. ID No: 12);
¡¡¡¡¡¡ AtrbohD-specific primers, ML1220, 5¡⁻-ATTACAAGCACCAAACCAG-3¡⁻ (SEQ. ID No: 13) and ML1221, 5¡⁻-
TGCCAAGCCATAACATCA-3¡⁻ (SEQ. ID No: 14); ¡¡¡¡¡¡AtrbohF-specific primers, ML1222, 5¡⁻-CTGCGGTTTCGCCATTC-3¡⁻ (SEQ. ID No: 15) and ML1223, 5¡⁻-TGTTTCGTCGGCTCTG-3¡⁻ (SEQ. ID No: 16).
¡¡¡¡¡¡ACBP2 full-length cDNA: 
attaaaaagt cttaacgccc acaacgaaaa ggaacttctc tttttgtttg gtgactgaat
cggtggaaaa caacacaagt ttgttccttt ttgtctctcc agatcccttg gcttccttct
tcttcttcat cctcctcgtg agactctgtg ttgaaatttc tctagggttt taattatcgg
cgttggtttt tcgtttttga gaatttgatt tcgtttttag aaataatggg tgattgggct
caacttgctc agtctgtgat cctaggtttg attttctctt accttctcgc taagctgatc
tcgatcgtcg tcacgtttaa agaagacaat ctctctctta ctcgtcaccc agaggagtct
caattggaaa ttaaaccgga aggagttgac tcgcgacgtc tcgattcctc ttgcggtggt
ttcggtggtg aggcggattc gcttgtggcg gagcagggta gctcccgaag tgatagcgtt
gccggtgacg acagtgagga agatgatgat tgggaaggcg tcgagagcac ggaactcgat
gaggctttta gcgccgccac tctttttgtg actaccgccg ccgcagatcg gctttcgcag
aaggtaccga gtgatgtgca gcagcagctt tacggattgt ataagattgc tacggaaggg
ccgtgtactg ctcctcagcc atcagctctc aaaatgactg ctcgtgccaa gtggcaagca
tggcagaaac tgggagctat gccacctgaa gaggcgatgg agaagtatat tgagattgtc
actcagcttt acccaacttg gttagacggt ggcgtgaaag ccggaagtcg gggtggggat
gatgcagcct ccaactcaag aggaaccatg ggaccagttt ttagctcttt ggtttatgat
gaggagtccg aaaatgagtt gaagattgat gccatacacg gatttgctag agaaggagaa
gtcgagaatt tactgaaaag cattgaaagc ggcattcctg taaatgcaag ggacagtgaa
ggtcgcacac cattgcactg ggctatagac cgtggccacc tcaacatcgc caaagttctg
gtcgataaga acgccgatgt gaatgctaag gacaacgaag gccaaacccc tttgcattat
gctgttgtat gcgacagaga agctatcgcc gagtttctgg ttaaacagaa cgcaaacaca
gccgctaaag atgaggatgg aaactctccc cttgatctct gtgaatcaga ctggccctgg
atccgagatt ctgcaaagca ggcagactaa acaaatacta accacgtctt ctctaaatcc
gcaatgtata tgatcaaatg acttagtaag aagcttttct ttactttaaa ctttctttcc
atccctacca catcactggg atgttccaac actatatcac ttggatgtta ccaagtcttg
ttctttgatt tctttcttct tacattttaa caatcttttg ttcctttagg ctttatgata
tgtcgtaacc ggttttgttg gtttgctata aatacacaca tatagaca
(SEQ. ID NO: 3)
¡¡¡¡¡¡
¡¡¡¡¡¡caMV 35S promoter-specific forward primer 35SB, 5¡⁻-CAATCCCACTATCCTTCGCAAGAC C-3¡⁻ (SEQ. ID NO: 5).
¡¡¡¡¡¡Amino acid sequence of ACBP2: 
MGDWAQLAQSVILGLIFSYLLAKLISIVVTFKEDNLSLTRHPEE
SQLEIKPEGVDSRRLDSSCGGFGGEADSLVAEQGSSRSDSVAGDDSEED
DDWEGVESTELDEAFSAATLFVTTAAADRLSQKVPSDVQQQLYGLYKI
ATEGPCTAPQPSALKMTARAKWQAWQKLGAMPPEEAMEKYIEIVTQLY
PTWLDGGVKAGSRGGDDAASNSRGTMGPVFSSLVYDEESENELKIDAIH
GFAREGEVENLLKSIESGIPVNARDSEGRTPLHWAIDRGHLNIAKVLVDK
NADVNAKDNEGQTPLHYAVVCDREAIAEFLVKQNANTAAKDEDGNS
PLDLCESDWPWIRDSAKQAD
(SEQ. ID NO: 6)
¡¡¡¡¡¡primers ML805: -1704 to -1677 in ACBP2 5¡⁻-flanking sequence 5¡⁻-CTGGATCCAGGAGTCAGCGTCGTATGTC-3¡⁻(SEQ. ID No: 7) and
ML806: 121-96 in ACBP2 gDNA 5¡⁻-TCCCCGGGGTGACGAGTAAGAGAGAG-3¡⁻ (SEQ. ID No: 8)

The nucleotide sequence of ACBP2pro:GUS fusion: 
ttcttttcaaaagcttcctctggaaacaggagtcagcgtcgtatgtctatcccctggtgttgtcctaacaaatgttgtgagt
attagcttcacttcatttcgttacaa
gtgcaggattcctctgttttctcagaaattcattgcgttgaactgcctatttctttccgaaatttacaggccagggatctatc
caggattcttcaagctctttacgca
gtgataccttatttcatatttcaccccaagaaggttgtagaagttctctattctcggccacagatcctcagattccagagta
ctgggaaacactaaaaaacgatg
 
attggcctgtttgcccattcatctctcaagattgccgccctgcaaatccttccgaagaagcacacaacacagaaactgcacag
agagtgtggaaaaagacgt
tagagctggtgggtcttcctctcgatgcagttgagaagctcatagaaggggaaaatatccaatgccggtatggagcacaacac
gaatagtctttcaaaattac
cacaggttaagtgacccattacagatcaaagggtaggtaattgagaaaatatcttttttttttgtttccttgtattaatctac
acgatacagtggggaatgaatcccc
caggcatgtagtttgcttgagaatgtttgattgttggataaaagtcaagctttagctaccattccattgcttttacttacaag
tcttgttcaagtatttggttagtgtgtc
ttggagttattaagatgttcagtagtagagtatggtagcctgggttggtgggtagtttcttgtttgagtttagggtattttcg
taattttctttattgtttgtggagtcac
gttgctcatatatagttatagggatatatatcaattagagtcatcatctctcttactctcatggtgacaaagtcacaaaagtg
ataaactctcctttccactttctaatg
tttcttgcttaaatgagtattccatgtaaaaatgattcactcactcgtatgggtgggttgggttttctcttcatgagatgctc
atttgagcaagcaatgtttggaagtg
ggactatttaatatcatgtatgagttttatcttttgttataagaaaaaaagttgggatttcatttcgtgcccacttcacgtg
aatctaatgccttcaagtttgtggtcta
ccttatttctcttatggatacatgtcaatttacgtgtcgtatgagtcaagcctttaatcatcccaacaccatgcacgtcttc
tagatttgttttcgaccacgattccac
cacaaaatttgacattgttattgaattcattaactttttacgtgttattgtgaaatatttatttaatttgttggtaaaagg
agcaaaaaagattagggtacaacacgtc
gacttcttccccaattagacccatatgtgatctgtcgtcactcgtcgccaagtttttttatgtgtcgtcttttaaactttgat
ccaattcattaaaaagtcttaacgccc
acaacgaaaaggaacttctctttttgtttggtgactgaatcggtggaaaacaacacaagtttgttcctttttgtctctccaga
tcccttggcttccttcttcttcttcat
cctcctcgtgagactctgtgttgaaatttctctagggtttaattatcggcgttggtttttcgttttgagaatttgatttcg
tttttagaaataatgggtgattgggctca
acttgctcagtctgtgatcctaggtttgatttctcttaccttctcgctaagctgatctcgatcgtcgtcacgtttaaagaag
acaatctctctcttactcgtcacccc
gggtacggtcagtcccttatgttacgtcctgtagaaaccccaacccgtgaaatcaaaaaactcgacggcctgtgggcattcag
tctggatcgcgaaaactgt
ggaattgatcagcgttggtgggaaagcgcgttacaagaaagccgggcaattgctgtgccaggcagttttaacgatcagttcgc
cgatgcagatattcgtaatt
atgcgggcaacgtctggtatcagcgcgaagtctttataccgaaaggttgggcaggccagcgtatcgtgctgcgtttcgatgcg
gtcactcattacggcaaag
tgtgggtcaataatcaggaagtgatggagcatcagggcggctatacgccatttgaagccgatgtcacgccgtatgttattgcc
gggaaaagtgtacgtatca
ccgtttgtgtgaacaacgaactgaactggcagactatcccgccgggaatggtgattaccgacgaaaacggcaagaaaaagcag
tcttacttccatgatttctt
taactatgccggaatccatcgcagcgtaatgctctacaccacgccgaacacctgggtggacgatatcaccgtggtgacgcatg
tcgcgcaagactgtaacc
acgcgtctgttgactggcaggtggtggccaatggtgatgtcagcgttgaactgcgtgatgcggatcaacaggtggttgcaact
ggacaaggcactagcgg
gactttgcaagtggtgaatccgcacctctggcaaccgggtgaaggttatctctatgaactgtgcgtcacagccaaaagccaga
cagagtgtgatatctaccc
gcttcgcgtcggcatccggtcagtggcagtgaagggccaacagttcctgattaaccacaaaccgttctactttactggctttg
gtcgtcatgaagatgcggac
ttacgtggcaaaggattcgataacgtgctgatggtgcacgaccacgcattaatggactggattggggccaactcctaccgtac
ctcgcattacccttacgctg
aagagatgctcgactgggcagatgaacatggcatcgtggtgattgatgaaactgctgctgtcggctttaacctctctttaggc
attggtttcgaagcgggcaa
caagccgaaagaactgtacagcgaagaggcagtcaacggggaaactcagcaagcgcacttacaggcgattaaagagctgatag
cgcgtgacaaaaacc
acccaagcgtggtgatgtggagtattgccaacgaaccggatacccgtccgcaagtgcacgggaatatttcgccactggcggaa
gcaacgcgtaaactcga
cccgacgcgtccgatcacctgcgtcaatgtaatgttctgcgacgctcacaccgataccatcagcgatctctttgatgtgctgt
gcctgaaccgttattacggat
ggtatgtccaaagcggcgatttggaaacggcagagaaggtactggaaaaagaacttctggcctggcaggagaaactgcatcag
ccgattatcatcaccga
atacggcgtggatacgttagccgggctgcactcaatgtacaccgacatgtggagtgaagagtatcagtgtgcatggctggata
tgtatcaccgcgtctttgat
cgcgtcagcgccgtcgtcggtgaacaggtatggaatttcgccgattttgcgacctcgcaaggcatattgcgcgttggcggtaa
caagaaagggatcttcact
cgcgaccgcaaaccgaagtcggcggcttttctgctgcaaaaacgctggactggcatgaacttcggtgaaaaaccgcagcaggg
 
aggcaaacaatga
(SEQ. ID No: 9)
 Amino acid sequence of ACBP2pro:GUS fusion:

MLRPVETPTREIKKLDGLWAFSLDRENCGIDQRWWESALQESRAIAVPGSFNDQFADADIRNYAG
NVWYQREVFIPKGWAGQRIVLRFDAVTHYGKVWVNNQEVMEHQGGYTPFEADVTPYVIAGKSV
RITVCVNNELNWQTIPPGMVITDENGKKKQSYFHDFFNYAGIHRSVMLYTTPNTWVDDITVVTHV
AQDCNHASVDWQVVANGDVSVELRDADQQVVATGQGTSGTLQVVNPHLWQPGEGYLYELCVT
AKSQTECDIYPLRVGIRSVAVKGQQFLINHKPFYFTGFGRHEDADLRGKGFDNVLMVHDHALMD
WIGANSYRTSHYPYAEEMLDWADEHGIVVIDETAAVGFNLSLGIGFEAGNKPKELYSEEAVNGET
QQAHLQAIKELIARDKNHPSVVMWSIANEPDTRPQVHGNISPLAEATRKLDPTRPITCVNVMFCDA
HTDTISDLFDVLCLNRYYGWYVQSGDLETAEKVLEKELLAWQEKLHQPIIITEYGVDTLAGLHSM
YTDMWSEEYQCAWLDMYHRVFDRVSAVVGEQVWNFADFATSQGILRVGGNKKGIFTRDRKPKS
AAFLLQKRWTGMNFGEKPQQGGKQ
(SEQ. ID No: 9)

## Claims

1. Use of *Arabidopsis thaliana* acyl-Coenzme A-binding protein 2 (ACBP2) according to SEQ ID NO: 6 or peptide sequences having at least 90% amino acid identity to SEQ ID NO: 6 and having Arabidopsis thaliana acyl-Coenzme A-binding protein 2 activity to enhance drought tolerance relative to an unmodified plant in a transgenic plant or a transgenic plant cell genetically engineered to express ACBP2 according to SEQ ID NO: 6 or peptide sequences having at least 90% amino acid identity to SEQ ID NO: 6 and having Arabidopsis thaliana acyl-Coenzme A-binding protein 2 activity in an amount effective to enhance drought tolerance relative to an unmodified plant.

2. Use of ACBP2 according to claim 1, wherein the transgenic plant or transgenic plant cell is of a solanaceous plant species, optionally tomato, or a grain crop or cotton.

3. A method of obtaining enhanced drought tolerance in a plant or plant cell comprising: genetically engineering the plant or plant cell to express *Arabidopsis thaliana* acyl-Coenzme A-binding protein 2 (ACBP2) according to SEQ ID NO: 6 or peptide sequences having at least 90% amino acid identity to SEQ ID NO: 6 and having Arabidopsis thaliana acyl-Coenzme A-binding protein 2 activity in an amount effective to provide drought tolerance relative to a non-genetically engineered plant.

4. The method of claim 3, wherein the plant cell is of a solanaceous plant species, or
wherein the plant cell is of a grain crop, or
wherein the plant cell is from a plant selected from the group consisting of a tomato, cotton and rice.

5. The method of claim 4, wherein the ACBP2 is Arabidopsis ACBP2.

6. A method of obtaining a plant part having drought tolerance, comprising: obtaining a plant part genetically modified to express *Arabidopsis thaliana* acyl-Coenzme A-binding protein 2 (ACBP2) according to SEQ ID NO: 6 or peptide sequences having at least 90% amino acid identity to SEQ ID NO: 6 and having Arabidopsis thaliana acyl-Coenzme A-binding protein 2 activity; and growing the plant part under conditions where it is exposed to drought stress sufficient to be growth-inhibiting to a native plant of the same type.

7. The method of claim 6, wherein obtaining the plant part comprises growing the plant part from a seed, or
wherein obtaining the plant part comprises obtaining a plant cutting, or
wherein the plant part is in a plant.

8. The method of claim 6, wherein the drought stress is at least 15 days without water.

9. A method of screening for functional variants of Arabidopsis thaliana acyl-Coenzme A-binding protein 2 (ACBP2) according to SEQ ID NO: 6 or peptide sequences having at least 90% amino acid identity to SEQ ID NO: 6 wherein the functional variants have at least 90% amino acid identity to SEQ ID NO: 6 and have Arabidopsis thaliana acyl-Coenzme A-binding protein 2 activity, comprising: obtaining a plant cell genetically modified to express a candidate ACBP2 variant; growing the plant cell under conditions of drought stress for a duration that is sufficient to be growth-inhibiting to a native plant cell of the same type; observing whether the plant cell exhibits a reduction in growth inhibition; and, if so, identifying the candidate ACBP2 variant as functional.

10. The method of claim 9, further comprising regenerating the genetically modified plant cell containing the functional ACBP2 variant into a plant.

11. The method of claim 9, further comprising obtaining other plant cells genetically modified to express the functional ACBP2 variant.

## Patentansprüche

1. Verwendung des *Arabidopsis thaliana-*Acyl-Coenzym A-Bindungsproteins 2 (ACBP2) gemäß SEQ ID NO: 6 oder von Peptidsequenzen mit mindestens 90 % Aminosäureidentität mit SEQ ID NO: 6 und mit *Arabidopsis thaliana*-Acyl-Coenzym A-Bindungsprotein 2-Aktivität zum Verbessern der Trockenheitstoleranz relativ zu einer unmodifizierten Pflanze, in einer transgenen Pflanze oder einer transgenen Pflanzenzelle, die zum Exprimieren von ACBP2 gemäß SEQ ID NO: 6 oder von Peptidsequenzen mit mindestens 90 % Aminosäureidentität mit SEQ ID NO: 6 und mit *Arabidopsis thaliana*-Acyl-Coenzym A-Bindungsprotein 2-Aktivität, in einer zum Verbessern der Trockenheitstoleranz wirksamen Menge relativ zu einer unmodifizierten Pflanze genetisch verändert ist.

2. Verwendung von ACBP2 nach Anspruch 1, wobei die transgene Pflanze oder die transgene Pflanzenzelle von einer Spezies eines Nachschattengewächses stammt, optional der Tomate oder einer Getreidenutzpflanze oder von Baumwolle.

3. Verfahren zum Erzielen einer verbesserten Trockenheitstoleranz in einer Pflanze oder einer Pflanzenzelle, das Folgendes umfasst: genetisches Verändern der Pflanze oder Pflanzenzelle zum Exprimieren des *Arabidopsis thaliana*-Acyl-Coenzym A-Bindungsproteins 2 (ACBP2) gemäß SEQ ID NO: 6 oder von Peptidsequenzen mit mindestens 90 % Aminosäurenidentität mit SEQ ID NO: 6 und mit *Arabidopsis thaliana*-Acyl-Coenzym A-Bindungsprotein 2-Aktivität in einer zum Verbessern der Trockenheitstoleranz wirksamen Menge relativ zu einer genetisch unveränderten Pflanze.

4. Verfahren nach Anspruch 3, wobei die Pflanzenzelle von einer Spezies eines Nachschattengewächses stammt, oder
wobei die Pflanzenzelle von einer Getreidenutzpflanze stammt, oder
wobei die Pflanzenzelle von einer Pflanze stammt, ausgewählt aus der Gruppe bestehend aus Tomate, Baumwolle und Reis.

5. Verfahren nach Anspruch 4, wobei das ACBP2 *Arabidopsis*-ACBP2 ist.

6. Verfahren zum Erhalten eines Pflanzenteils mit Trockenheitstoleranz, das Folgendes umfasst: Erhalten eines genetisch modifizierten Pflanzenteils zum Exprimieren des *Arabidopsis thaliana*-Acyl-Coenzym A-Bindungsproteins 2 (ACBP2) gemäß SEQ ID NO: 6 oder von Peptidsequenzen mit mindestens 90 % Aminosäurenidentität mit SEQ ID NO: 6 und mit *Arabidopsis thaliana*-Acyl-Coenzym A-Bindungsprotein 2-Aktivität; und Wachsenlassen des Pflanzenteils unter Bedingungen, in denen er Trockenheitsbelastungen ausgesetzt ist, die ausreichend sind, um das Wachstum einer nativen Pflanze desselben Typs zu hemmen.

7. Verfahren nach Anspruch 6, wobei das Erhalten des Pflanzenteils das Wachsenlassen des Pflanzenteils aus einem Samen umfasst, oder
wobei das Erhalten des Pflanzenteils das Erhalten eines Pflanzenschnitts umfasst, oder wobei der Pflanzenteil sich in einer Pflanze befindet.

8. Verfahren nach Anspruch 6, wobei die Trockenheitsbelastungen mindestens 15 Tage ohne Wasser betragen.

9. Verfahren zum Screenen nach funktionalen Varianten des *Arabidopsis thaliana*-Acyl-Coenzym A-Bindungsproteins 2 (ACBP2) gemäß SEQ ID NO: 6 oder von Peptidsequenzen mit mindestens 90 % Aminosäurenidentität mit SEQ ID NO: 6, wobei die funktionalen Varianten mindestens 90 % Aminosäurenidentität mit SEQ ID NO: 6 und *Arabidopsis thaliana*-Acyl-Coenzym A-Bindungsprotein 2-Aktivität aufweisen, wobei das Verfahren Folgendes umfasst: Erhalten einer genetisch modifizierten Pflanzenzelle zum Exprimieren eines Kandidaten für eine ACBP2-Variante; Wachsenlassen der Pflanzenzelle unter Bedingungen von Trockenheitsbelastungen über eine Zeitdauer, die ausreichend ist, um das Wachstum einer nativen Pflanze desselben Typs zu hemmen; Beobachten, ob die Pflanzenzelle eine Reduzierung der Wachstumshemmung zeigt; und falls ja, Identifizieren des Kandidaten für eine ACBP2-Variante als funktional.

10. Verfahren nach Anspruch 9, das ferner das Regenerieren der die funktionale ACBP2-Variante enthaltenden, genetisch modifizierten Pflanzenzelle in eine Pflanze umfasst.

11. Verfahren nach Anspruch 9, das ferner das Erhalten weiterer genetisch modifizierter Pflanzenzellen zum Exprimieren der funktionalen ACBP2-Variante umfasst.

## Revendications

1. Utilisation de la protéine 2 se liant à l'acyl-coenzyme A d'*Arabidopsis thaliana* (ACBP2) selon SEQ ID n° 6 ou séquences peptidiques ayant au moins 90 % d'identité d'acides aminés à SEQ ID n° 6 et ayant une activité de protéine 2 se liant à l'acyl-coenzyme A d'Arabidopsis thaliana pour augmenter la tolérance à la sécheresse relativement à une plante non modifiée dans une plante transgénique ou une cellule de plante transgénique modifiée par génie génétique pour exprimer ACBP2 selon SEQ ID n° 6 ou des séquences peptidiques ayant au moins 90 % d'identité d'acides aminés à SEQ ID n° 6 et ayant une activité de protéine 2 se liant à l'acyl-coenzyme A d'Arabidopsis thaliana en une quantité efficace pour augmenter la tolérance à la sécheresse relativement à une plante non modifiée.

2. Utilisation d'ACBP2 selon la revendication 1, dans laquelle la plante transgénique ou la cellule de plante transgénique est d'une espèce de plante solanacée, éventuellement la tomate, ou une culture céréalière ou le coton.

3. Procédé d'obtention de tolérance à la sécheresse augmentée dans une plante ou une cellule de plante comprenant : la modification par génie génétique de la plante ou de la cellule de plante pour exprimer la protéine 2 se liant à l'acyl-coenzyme A d'*Arabidopsis thaliana* (ACBP2) selon SEQ ID n° 6 ou séquences peptidiques ayant au moins 90 % d'identité d'acides aminés à SEQ ID n° 6 et ayant une activité de protéine 2 se liant à l'acyl-coenzyme A d'Arabidopsis thaliana en une quantité efficace pour fournir la tolérance à la sécheresse relativement à une plante non modifiée par génie génétique.

4. Procédé selon la revendication 3, dans lequel la cellule de plante est d'une espèce de plante solanacée, ou
dans lequel la cellule de plante est d'une culture céréalière, ou
dans lequel la cellule de plante est d'une plante choisie dans le groupe constitué par une tomate, le coton ou le riz.

5. Procédé selon la revendication 4, dans lequel l'ACBP2 est l'ACBP2 d'Arabidopsis.

6. Procédé d'obtention d'une partie de plante ayant une tolérance à la sécheresse, comprenant : l'obtention d'une partie de plante modifiée par génie génétique pour exprimer la protéine 2 se liant à l'acyl-coenzyme A d'*Arabidopsis thaliana* (ACBP2) selon SEQ ID n° 6 ou des séquences peptidiques ayant au moins 90 % d'identité d'acides aminés à SEQ ID n° 6 et ayant une activité de protéine 2 se liant à l'acyl-coenzyme A d'Arabidopsis thaliana ; et la croissance de la partie de plante dans des conditions où elle est exposée à un stress de sécheresse suffisante pour inhiber la croissance d'une plante native du même type.

7. Procédé selon la revendication 6, dans lequel l'obtention de la partie de plante comprend la croissance de la partie de plante à partir d'une graine, ou
dans lequel l'obtention de la partie de plante comprend l'obtention d'une coupure de plante, ou
dans lequel la partie de plante est dans une plante.

8. Procédé selon la revendication 6, dans lequel le stress de sécheresse est d'au moins 15 jours sans eau.

9. Procédé de criblage de variants fonctionnels de la protéine 2 se liant à l'acyl-coenzyme A d'*Arabidopsis thaliana* (ACBP2) selon SEQ ID n° 6 ou de séquences peptidiques ayant au moins 90 % d'identité d'acides aminés à SEQ ID n° 6 et ayant une activité de protéine 2 se liant à l'acyl-coenzyme A d'Arabidopsis thaliana, comprenant : l'obtention d'une cellule de plante modifiée par génie génétique pour exprimer un variant d'ACBP2 candidat ; la croissance de la cellule de plante dans des conditions de stress de sécheresse pour une durée qui est suffisante pour inhiber la croissance d'une cellule de plante native du même type ; l'observation si la cellule de plante présente une réduction d'inhibition de croissance ; et, s'il en est ainsi, l'identification du variant d'ACBP2 candidat comme fonctionnel.

10. Procédé selon la revendication 9, comprenant en outre la régénération de la cellule de plante modifiée par génie génétique contenant le variant d'ACBP2 fonctionnel en une plante.

11. Procédé selon la revendication 9, comprenant en outre d'autres cellules de plante modifiées par génie génétique pour exprimer le variant d'ACBP2 fonctionnel.
